# EUROPEAN PATENT SPECIFICATION

(11) **EP 4 025 259 B1**
(45) Date of publication and mention of the grant of the patent: **30.10.2024**
(21) Application number: 20767545.5
(22) Date of filing: 03.09.2020
(51) Int. Cl.: A61K 49/10, C07D 471/08, C07F 13/00

(54) **MANGANESE CHELATE ISOMERS**
MANGANCHELATISOMERE
ISOMÈRES DE CHÉLATE DE MANGANÈSE

(30) Priority: 03.09.2019 US 201962895121 P
(43) Date of publication of application: 13.07.2022
(73) Proprietor: GE Healthcare AS, 0485 Oslo (NO)
(72) Inventor: BALES, Brian, C, Niskayuna, New York 12309-1027 (US); RISHEL, Michael, James, Niskayuna, New York 12309-1027 (US); DINN, Sean, R, Niskayuna, New York 12309-1027 (US)
(74) Representative: Smart, Jessica Ann
(86) International application number: PCT/EP2020/074645
(87) International publication number: WO 2021/043926

(56) References cited:
- WO-A1-2017/220610
- ZOLTÁN GARDA ET AL: "Effect of the Nature of Donor Atoms on the Thermodynamic, Kinetic and Relaxation Properties of Mn(II) Complexes Formed With Some Trisubstituted 12-Membered Macrocyclic Ligands", FRONTIERS IN CHEMISTRY, vol. 6, 13 August 2018 (2018-08-13), XP055750721, DOI: 10.3389/fchem.2018.00232
- MAURO BOTTA ET AL: "Mn(II) compounds as an alternative to Gd-based MRI probes", FUTURE MEDICINAL CHEMISTRY, vol. 11, no. 12, 1 June 2019 (2019-06-01), GB, pages 1461 - 1483, XP055750733, ISSN: 1756-8919, DOI: 10.4155/fmc-2018-0608

## Description

### TECHNICAL FIELD OF THE INVENTION

The invention relates to isomers of chelate compounds and their use as contrast agents in magnetic resonance imaging (MRI) procedures.

### DESCRIPTION OF THE RELATED ART

MRI is a medical imaging technique in which areas of the body are visualized via the nuclei of selected atoms, especially hydrogen nuclei. The MRI signal depends upon the environment surrounding the visualized nuclei and their longitudinal and transverse relaxation times, T1 and T2. Thus, in the case when the visualized nucleus is a proton, the MRI signal intensity will depend upon factors such as proton density and the chemical environment of the protons. Contrast agents can be used in MRI to improve the imaging contrast. They work by effecting the T1 , T2 and/or T2* relaxation time and thereby influence the contrast in the images.

It is known that paramagnetic contrast agents can modify T1 , T2 and/or T2* relaxation times and this effect can be optimized by structural modification of the paramagnetic metal chelate. Of particular importance is the presence and residence time of a water molecule bound to the paramagnetic ion and the rotational correlation time of the contrast agent. The presence and residence time of a water molecule, bound to the paramagnetic ion, can be modulated by the choice of paramagnetic ion and the chelating moiety. The rotational correlation time can be modulated by varying the size of the contrast agent.

The solubility of the paramagnetic chelate in water is also an important factor when they are used as contrast agents for MRI because they are administered to patients in relatively large doses. A highly water-soluble paramagnetic chelate requires a lower injection volume, is thus easier to administer to a patient and causes less discomfort. Water-soluble paramagnetic chelates, i.e. complexes of a chelator and a paramagnetic metal ion are well known - for instance the commercially-available gadolinium chelates Omniscan^{™} (GE Healthcare), Dotarem^{™} (Guerbet), Gadavist^{™} (Bayer) and Magnevist^{™} (Bayer). Because of their low molecular weight they rapidly distribute into the extracellular space (i.e. the blood and the interstitium) when administered into the vasculature. They are also cleared relatively rapidly from the body.

A key property of MRI chelate compounds is for the paramagnetic ion to be retained as far as possible within the chelate structure. Paramagnetic ion released from the chelate in vivo can interfere with biological pathways and potentially induce toxicity. The ability of a chelate to retain the paramagnetic ion (also referred to herein as stability) is also a property that can be modulated by structural design of the cheland moiety. Of particular interest is the kinetic stability, measured as a dissociation half-life, which indicates the degree of inertia towards altered chemical surroundings (i.e. endogenous ions).

As can be appreciated from the commercially-available agents and the focus of the prior art, gadolinium is the most widely used paramagnetic metal ion for MRI chelates, which is due to its favorable relaxivity properties. The concept of "relaxivity" of an MRI agent is well known to those of skill in the art and refers to the ability of magnetic compounds to increase the relaxation rates of the surrounding water proton spins. Relaxivity is used to improve the contrast of an MR image, and to study tissue specific areas where the contrast agent better diffuses or to perform functional MRI. The relaxivity of MRI agents depends on the molecular structure and kinetic of the complex. Relaxivity depends on the temperature, field strength, and substance in which the contrast agent is dissolved.

Stability of the paramagnetic ion within the chelate structure is desirable for gadolinium chelates when used as contrast agents. There is therefore a desire to identify new gadolinium chelates having higher levels of stability, particularly within physiological environments.

The manganese(II) ion is a paramagnetic species with a high spin number and a long electronic relaxation time and the potential of a manganese(ll) based high relaxivity contrast agent has been reported in the literature (Toth, E, Advances in Inorganic Chemistry, 2009, 61 (09), 63-129). Certain manganese(ll) chelates developed to date have however proved to be much less stable compared to corresponding gadolinium chelates. For example, the manganese chelate of DOTA (MnDOTA) is several hundred times less stable compared to the corresponding gadolinium complex Gd-DOTA (Drahos, B; Inorganic Chemistry, 2012(12), 1975-1986).

WO2011/073371 to Andreas Meijer published June 23, 2011 describes a molecular design that favors high chelate stability and a high relaxivity. This makes these compounds very suitable for use as MRI contrast agents. An exemplary compound of WO2011/073371 has the following structure:

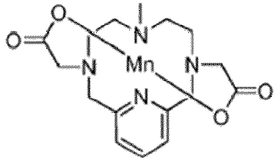

WO2017/220610 to Andreas Meijer et al., published December 28, 2017, describes manganese chelates that are suitable for use as contrast agents, and provide superior properties to other known manganese-based contrast agents. The manganese chelates of WO2017/220610 include compounds of Formula (1): 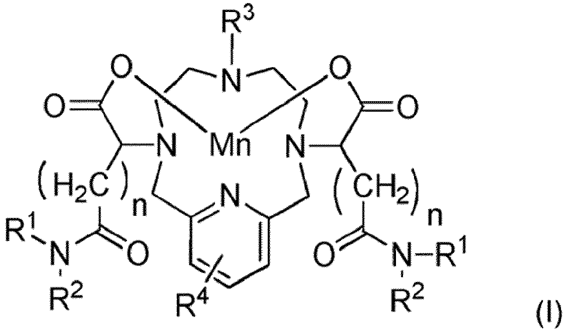 or a salt or solvate thereof, wherein:
each R¹ is independently selected from the group comprising C₁₋₂₀ hydroxyalkyl, C₁₋₆ alkyl, C₃₋₆ aryl optionally-substituted with one or more substituents selected from halo and -C(=O)-NH-C₁₋₆ hydroxyalkyl, or a carbohydrate moiety;
each R² is independently selected from the group comprising C₁₋₂₀ hydroxyalkyl, C₁₋₆ alkyl or hydrogen;
R³ is selected from the group comprising C₁₋₃ alkyl or -(CH₂)ₘ-C(=0)-NR⁵R⁶ wherein m is an integer from 2-5, and R⁵ and R⁶ are as respectively defined for R¹ and R²;
R⁴ represents 0-3 substituents selected from the group comprising hydroxy, C₁₋₆ alkyl and C₁₋₆ hydroxyalkyl; and,
each n is an integer from 0-4;
and wherein the compound of Formula I comprises at least two hydroxy groups.

There remains a need to develop manganese contrast agents having stability that are suitable for use as contrast agents.

### SUMMARY OF THE INVENTION

The present invention relates to compound of formula IA: 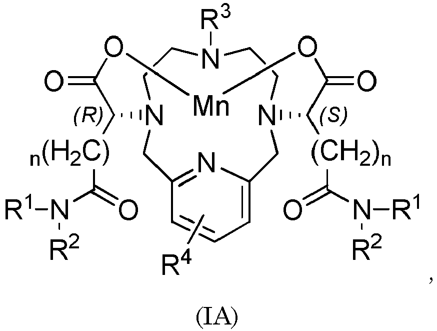 or a salt or solvate thereof. In the above formula, each R¹ is independently selected from C₁₋₂₀ hydroxyalkyl, C₁₋₆ alkyl, C₃₋₆ aryl optionally-substituted with one or more substituents selected from halo and -C(=O)-NH-C₁₋₆ hydroxyalkyl, or a carbohydrate moiety. Each R² is independently selected from C₁₋₂₀ hydroxyalkyl, C₁₋₆ alkyl or hydrogen. R³ is selected from C₁₋₃ alkyl or -(CH₂)ₘ-C(=0)- NR⁵R⁶, wherein m is an integer from 2-5, wherein R⁵ and R⁶ are independently selected from hydrogen, from C₁₋₂₀ hydroxyalkyl, C₁₋₆ alkyl, C₃₋₆ aryl optionally-substituted with one or more substituents selected from halo and -C(=O)-NH-C₁₋₆ hydroxyalkyl, or a carbohydrate moiety. R⁴ represents 0-3 substituents selected from hydroxy, C₁₋₆ alkyl and C₁₋₆ hydroxyalkyl. Each n is an integer from 0-4.

In one embodiment, the invention involves composition comprising a compound of formula IA, or a salt or solvate thereof, and a pharmaceutically acceptable excipient. In one embodiment, the composition lacks detectable amounts of a compound of Formula IB: 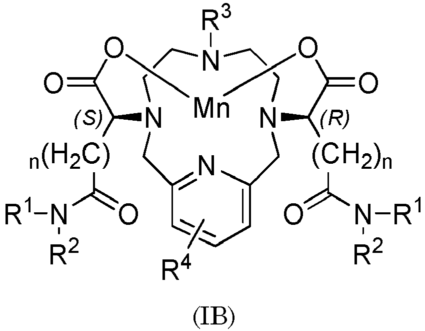 where each R¹ is independently selected from C₁₋₂₀ hydroxyalkyl, C₁₋₆ alkyl, C₃₋₆ aryl optionally-substituted with one or more substituents selected from halo and -C(=O)-NH-C₁₋₆ hydroxyalkyl, or a carbohydrate moiety. Each R² is independently selected from C₁₋₂₀ hydroxyalkyl, C₁₋₆ alkyl or hydrogen. R³ is selected from C₁₋₃ alkyl or -(CH₂)ₘ-C(=0)- NR⁵R⁶, wherein m is an integer from 2-5, wherein R⁵ and R⁶ are independently selected from hydrogen, from C₁₋₂₀ hydroxyalkyl, C₁₋₆ alkyl, C₃₋₆ aryl optionally-substituted with one or more substituents selected from halo and -C(=O)-NH-C₁₋₆ hydroxyalkyl, or a carbohydrate moiety. R⁴ represents 0-3 substituents selected from hydroxy, C₁₋₆ alkyl and C₁₋₆ hydroxyalkyl. Each n is an integer from 0-4.

In one embodiment, the invention involves a method of imaging a patient comprising administering the contrast agent, or contrast agent composition, followed by acquiring an MRI image of the patient.

In one embodiment, the invention involves a method of making the imaging agent as described above. The method may involve enantioselective synthesis, or isomerization from compounds of Formula (1A) to compounds of Formula (1B).

### BRIEF DESCRIPTION OF THE DRAWINGS

Fig. 1 shows the predicted stereoisomers of Mn Chelate-5.
Fig. 2 shows the HPLC chromatographs for (a) Mn Chelate-5 synthesized without stereochemical control, (b) (*R*,*S*)-Mn Chelate-5, (c) (R,R)-Mn Chelate-5, (d) (*S*,*S*)-Mn Chelate-5, and (e) (*S,R*)-Mn Chelate-5.
Fig. 3 Percent dechelation of Mn Chelate-5 stereoisomers in human serum
Fig. 4 shows the % Mn Chelate-5 remaining as a function of time for (a) (R,R)-Mn Chelate-5 (diamonds), (b) (*S,S*)-Mn Chelate-5 (squares), (c) (*S,R*)-Mn Chelate-5 (circles), and (d) (*R*,*S*)-Mn Chelate-5 (triangles) when incubated in the presence of xs. ZnCl₂ at pH = 4 and 40 °C.
Fig. 5 shows %ID (injected dose)/organ 7 d post injection of a 0.62 mmol Mn Chelate-5/kg dose containing ~30 µmol Mn-54 Chelate-5 (a) synthesized without stereochemical control (solid bars), (b) a 1:1 mixture of *R,R* & *S,S*)-Mn Chelate-5 isomers (hashed bars), and (c) (*R*,*S*)-Mn Chelate-5 (checkered bars).

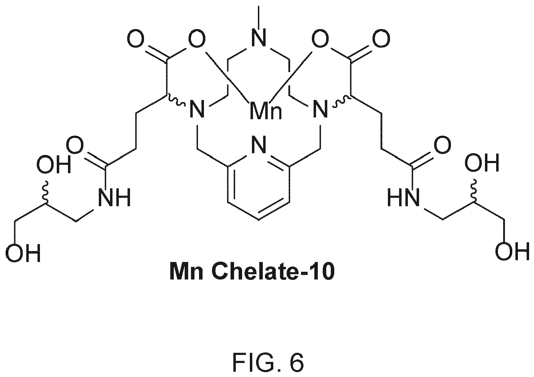

Fig. 6 shows the structure of Mn Chelate-10.

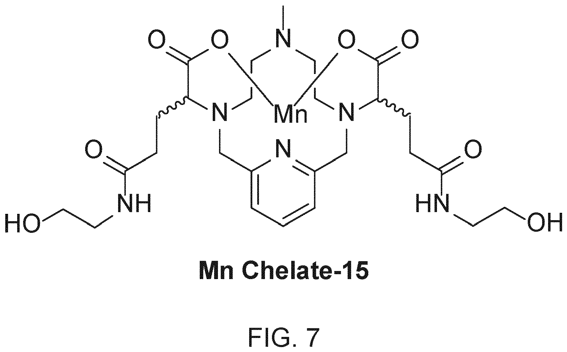

Fig. 7 shows the structure of Mn Chelate-15.

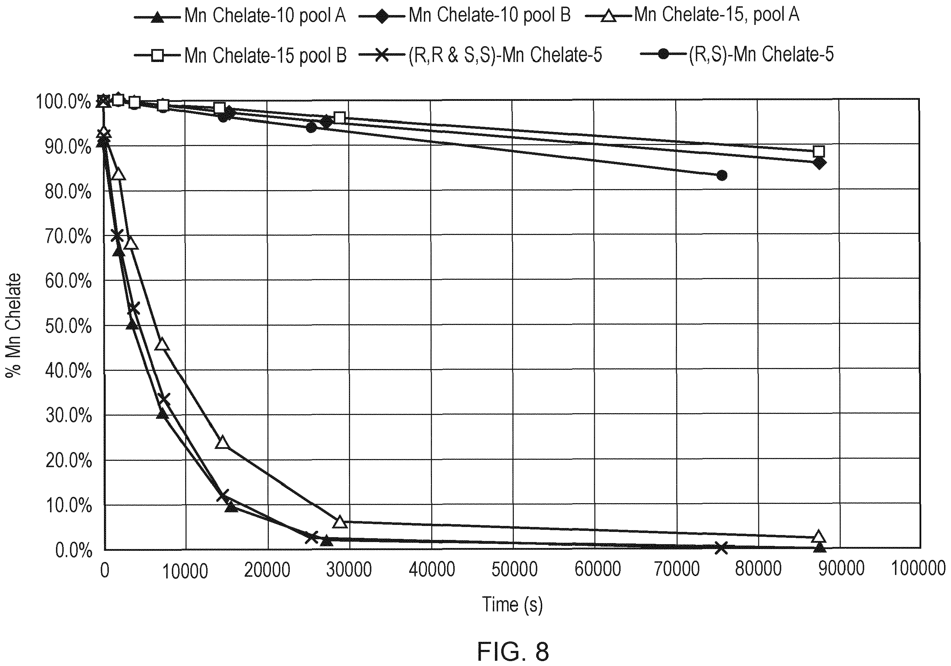

Fig. 8 shows the % Mn chelate remaining as a function of time for the (*R*,*S*)-Mn Chelate-5 (circles), a 1:1 mixture of *(R,R* & *S,S*)-Mn Chelate-5 isomers (X), Mn Chelate-10 isomer pool A (triangles), and Mn Chelate-10 isomer pool B (diamonds).
Fig. 9 shows the synthetic route to isomerically pure chelates according to embodiments of the invention.
Fig. 10 is an example chromatogram showing the separation of manganese-containing proteins from intact chelate.

### DETAILED DESCRIPTION OF THE INVENTION

To more clearly and concisely describe and point out the subject matter of the claimed invention, definitions and exemplary embodiments are provided hereinbelow for specific terms used throughout the present specification and claims. Any exemplification of specific terms herein should be considered as a non-limiting example.

The terms "comprising" or "comprises" have their conventional meaning throughout this application and imply that the agent or composition must have the essential features or components listed, but that others may be present in addition. The term 'comprising' includes as a preferred subset "consisting essentially of' which means that the composition has the components listed without other features or components being present.

A "salt" according to the invention, include physiologically acceptable acid addition salts such as those derived from mineral acids, for example hydrochloric, hydrobromic, phosphoric, metaphosphoric, nitric and sulphuric acids, and those derived from organic acids, for example tartaric, trifluoroacetic, citric, malic, lactic, fumaric, benzoic, glycollic, gluconic, succinic, methanesulphonic, and para- toluenesulphonic acids.

A suitable "solvate" according to the invention is selected from ethanol, water, saline, physiological buffer and glycol.

The term "alkyl", alone or in combination, means a straight-chain or branched-chain alkyl radical having the general formula CₙH₂ₙ₊₁. Examples of such radicals include methyl, ethyl, and isopropyl.

The term "hydroxyl" refers to the group -OH.

The term "hydroxyalkyl" refers to an alkyl group as defined above comprising one or more hydroxyl substituent(s) as defined above.

The term "aryl" refers to a functional group or substituent derived from an aromatic ring, usually an aromatic hydrocarbon, examples of which include phenyl and pyridyl. In one embodiment aryl groups of the present invention are aromatic 6-membered rings with between 0-3 heteroatoms selected from O, N and S.

The term "halogen" or "halo" means a substituent selected from fluorine, chlorine, bromine or iodine.

The term "hydroxy" refers to the group -OH.

The term "chelate moiety" refers to a substituent that is a metal chelate where the term "metal chelate" refers to a coordination complex wherein a metal ion is bonded to a surrounding array of molecules or anions comprised in a cheland. A "cheland" is defined herein as an organic compound capable of forming coordinate bonds with a paramagnetic metal ion through two or more donor atoms. In a typical cheland suitable for the present invention 2-6, and preferably 4-6, metal donor atoms are arranged such that 5- or 6-membered rings result (by having a non-coordinating backbone of either carbon atoms or non-coordinating heteroatoms linking the metal donor atoms). Examples of suitable donor atom types where the metal ion is a paramagnetic metal ion include amines, thiols, amides, oximes, and phosphines. In one embodiment the metal ion is manganese.

In one aspect the invention involves a compound of formula IA: 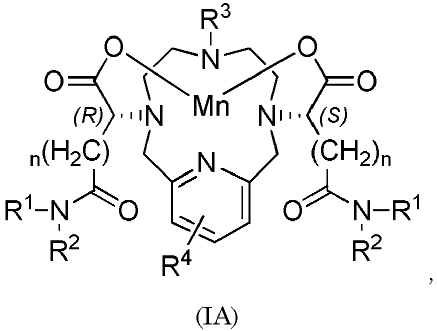 or a salt or solvate thereof. In this formula each R¹ is independently selected from C₁₋₂₀ hydroxyalkyl, C₁₋₆ alkyl, C₃₋₆ aryl optionally-substituted with one or more substituents selected from halo and -C(=O)-NH-C₁₋₆ hydroxyalkyl, or a carbohydrate moiety; each R² is independently selected from C₁₋₂₀ hydroxyalkyl, C₁₋₆ alkyl or hydrogen; R³ is selected from C₁₋₃ alkyl or -(CH₂)ₘ-C(=0)- NR⁵R⁶, wherein m is an integer from 2-5, wherein R⁵ and R⁶ are independently selected from hydrogen, from C₁₋₂₀ hydroxyalkyl, C₁₋₆ alkyl, C₃₋₆ aryl optionally-substituted with one or more substituents selected from halo and -C(=O)-NH-C₁₋₆ hydroxyalkyl, or a carbohydrate moiety; R⁴ represents 0-3 substituents selected from hydroxy, C₁₋₆ alkyl and C₁₋₆ hydroxyalkyl; and, each n is an integer from 0-4.

The following process illustrates the stereoselective synthesis of the *(R,R)* and (*S*,*S*) isomers of Mn Chelate-5. (*R,R*)-Mn Chelate-5 was synthesized from deprotected 2-arm cyclic chelate (Compound III) via bis-alkylated with (*S*)-5-benzyl 1-*tert*-butyl 2-(methylsulfonyloxy) pentanedioate to give protected (*R,R*)-Mn 2-arm C5 chelate (Compound V). Protected (*R,R*)-Mn 2-arm C5 chelate was then deprotected under acidic conditions, Mn was chelated, and aminoalcohol (*D-*glucamine) was coupled, yielding (*R,R*)-Mn Chelate-5. (*S*,*S*)-Mn Chelate-5 was synthesized from deprotected 2-arm cyclic chelate (Compound III) via bis alkylated with (*R*)-5-benzyl 1-*tert*-butyl 2-(methylsulfonyloxy) pentanedioate to give protected (*S*,*S*)-Mn 2-arm C5 chelate (Compound VI). (*S*,*S*)-Mn 2-arm C5 chelate was converted to (*S,S*)-Mn Chelate-5 using the approach described above for the synthesis of (*R,R*)-Mn Chelate-5.

The following process illustrates the stereoselective synthesis of the *(S,R)* and (*R,S*) isomers of Mn Chelate-5. (*S,R*)-Mn Chelate-5 and (*R,S*)-Mn Chelate-5 were synthesized from deprotected 2-arm cyclic chelate via mono-alkylation with (*S*)-5-benzyl 1-*tert*-butyl 2-(methylsulfonyloxy) pentanedioate followed by alkylation with (*R*)-5-benzyl 1-*tert*-butyl 2-(methylsulfonyloxy) pentanedioate to give (*R,S)*-Mn 2-arm C5 chelate (Compound VII). (*R*,*S*)-Mn 2-arm C5 chelate was then deprotected under acidic conditions and Mn was chelated to give a mixture of (*S,R*)-Mn 2-arm C5 chelate and (*R*,*S*)-Mn 2-arm C5 chelate. The mixture of (*S*,*R*)-Mn 2-arm C5 chelate and (*R*,*S*)-Mn 2-arm C5 chelate was then coupled with aminoalcohol (*D-*glucamine) to give a mixture of (*S*,*R*)-Mn Chelate-5 and (*R*,*S*)-Mn Chelate-5 that was separated via C¹⁸ chromatography. Additionally, (*R*,*S*)-Mn Chelate-5 chelate was synthesized by heating a mixture of (*S*,*R*)-Mn 2-arm C5 chelate and (*R*,*S*)-Mn 2-arm C5 chelate at 90 °C until isomerization of the (*S,R*)-Mn 2-arm C5 chelate to (*R*,*S*)-Mn 2-arm C5 chelate was complete and then the (*R*,*S*)-Mn 2-arm C5 chelate was coupled with aminoalcohol (*D*-glucamine) to give (*R*,*S*)-Mn Chelate-5. As can be appreciated by one skilled in the art, the approach described for the stereoselective synthesis of the isomers of the Mn Chelate-5 is applicable to the stereoselective synthesis of the compounds described by Formula IA and IB.

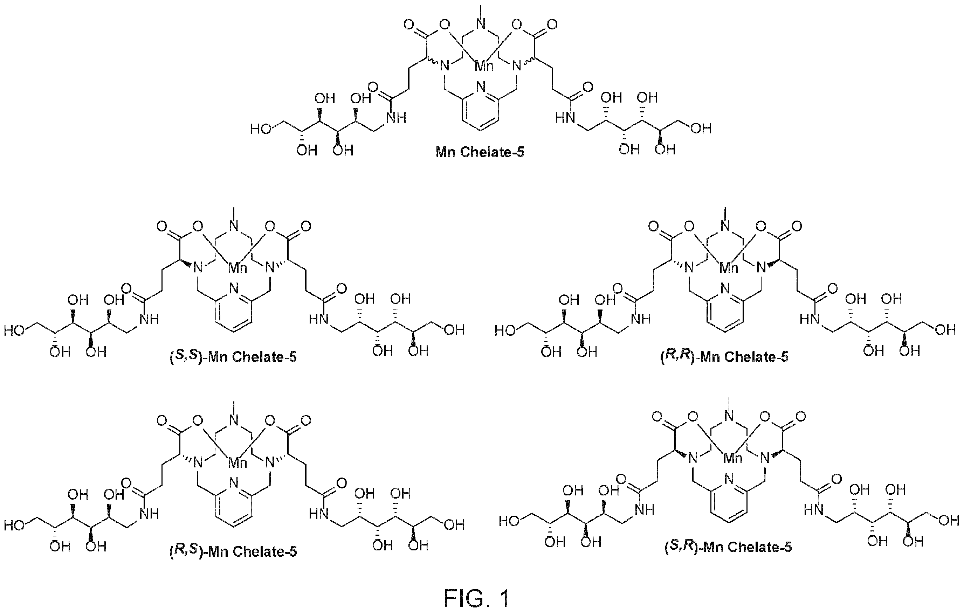

Mn Chelate-5 was synthesized without stereochemical control at the sites of connection to the macrocycle. In the absence of stereochemical control, 4 isomers were expected to be present as shown in Fig. 1. The *(S,R)* and (*R,S*) isomers can be interconverted via dechelation and rechelation of the metal. *R* and *S* designations represent the stereochemistry at the points of connection to the macrocycle.

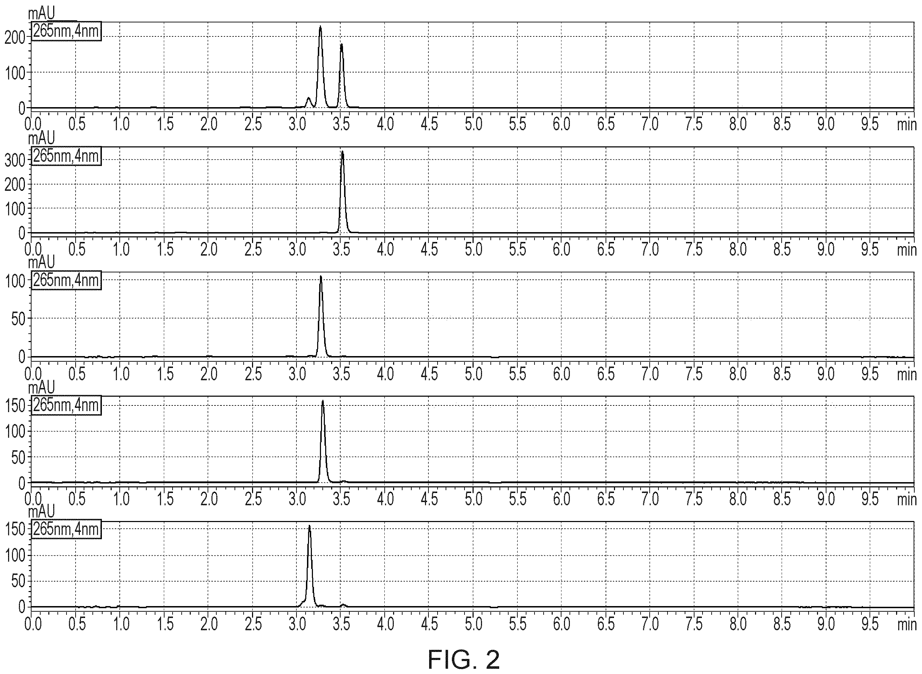

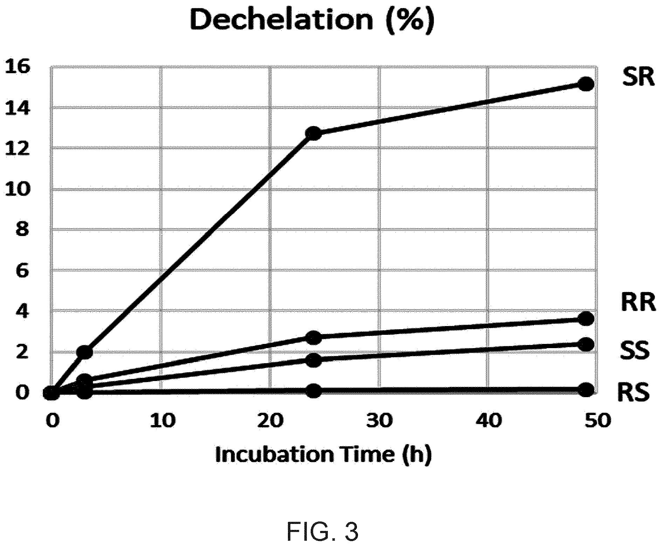

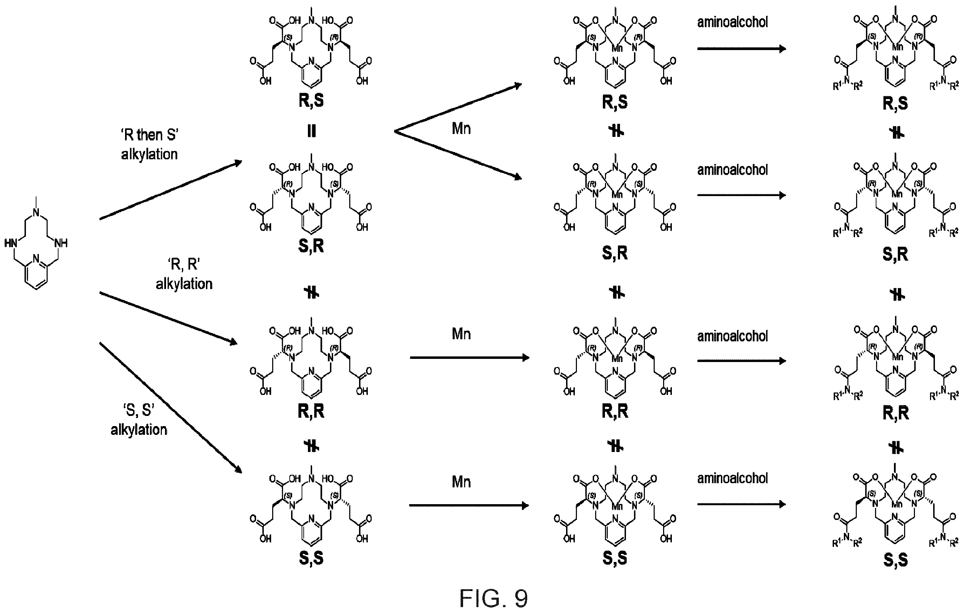

Fig. 2a shows the results from HPLC analysis of Mn Chelate-5 synthesized without stereochemical control. Subsequently, (*S*,*S*)-Mn Chelate-5 and (*R,R*)-Mn Chelate-5 were enantioselectivly synthesized as illustrated in Fig. 9. The results from HPLC analysis of (*R,R*)-Mn Chelate-5 are shown in Fig. 2c and the HPLC analysis of (*S*,*S*)-Mn Chelate-5 are shown in Fig. 2d. Enantioselective synthesis of (*R*,*S*)-Mn Chelate-5 and (*S,R*)-Mn Chelate-5 was performed as illustrated in Fig. 9. The results from HPLC analysis of (*R*,*S*)-Mn Chelate-5 are shown in Fig. 2b and the results from HPLC analysis of (*S*,*R*)-Mn Chelate-5 are shown in Fig. 2e.
The stability of Mn Chelate-5 to transmetallation with Zn was assessed with 2 mM Mn Chelate-5 at pH = 4 in the presence of 100 fold excess Zn (200 mM ZnCl₂ in 15 mM ammonium formate, pH = 4 at 40 °C. Fig. 3 shows the % Mn Chelate-5 remaining as a function of time for (*R,R)*-Mn Chelate-5 (diamonds), (*S*,*S*)-Mn Chelate-5 (open squares), (*S*,*R*)-Mn Chelate-5 (circles), and (*R*,*S*)-Mn Chelate-5 (triangles). Surprisingly, the data in Fig. 3 shows a significantly slower rate of transmetallation for (*R*,*S*)-Mn Chelate-5 isomer and a significantly faster rate of transmetallation for the (*S*,*R*)-Mn Chelate-5 isomer, suggesting a difference in the strength of Mn chelation as a function of chelate stereochemistry. Stability of Mn Chelate-5 stereoisomers in human serum was assessed *in vitro* by monitoring transfer of Mn from the chelate to blood proteins by a method employing size exclusion chromatography combined with online detection of manganese containing species by ICP-MS. Fig. 3 shows percent dechelation during the 2 days total incubation time at 37 oC. There is a significant difference between the (S,R)-Mn Chelate 5 isomer (upper curve, least stable) and the (R,S)-Mn Chelate 5 isomer (lower curve, most stable), in accordance with the results obtained in the Zn transmetallation study.

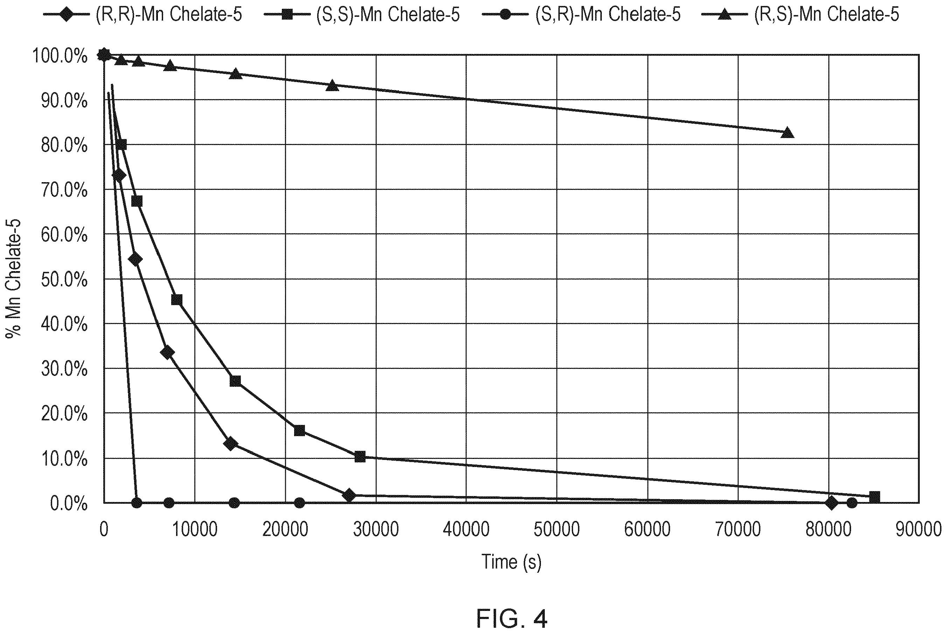

In vivo stability of the different isomer pools was assessed via a ⁵⁴Mn radioactive biodistribution as Mn released in vivo is known to be retained in brain, bone and liver. The amount of Mn released in vivo was assessed for Mn Chelate-5 synthesized without stereochemical control, a 1:1 mix of (*R,R*)-Mn Chelate-5 and (*S,S*)-Mn Chelate 5 isomers, and (*R*,*S*)-Mn Chelate-5 isomer pool B using ⁵⁴Mn labeled material added to non-radiolabeled material. The 1:1 mixture of (*R,R*)-Mn Chelate-5 and (*S*,*S*)-Mn Chelate-5 isomers was chosen as the 2 isomers were expected to have comparable stability based on their similar rates of transmetallation. Rats were dosed with 0.62 mmol Mn Chelate-5/kg containing ~30 µCi ⁵⁴Mn Chelate-5. The animals were sacrificed at 7 d post injection, organs of interest were collected, and the ⁵⁴Mn remaining in the organs was measured using a gamma counter. Fig. 4 is a plot showing %ID (injected dose)/organ 7 d post injection of a 0.62 mmol Mn Chelate-5/kg dose containing ~30 µmol ⁵⁴Mn Chelate-5. Bars containing hashed lines represent %ID/organ at or below the limit of detection for the study. The results of statistical comparison of the %ID for each organ are summarized as p-values using either a one-way ANOVA Games-Howell comparison or a 2-way t-test as appropriate are shown for each organ.

Surprisingly, the data in Fig. 4 shows significantly less Mn remaining in vivo at 7 d post injection for (*R*,*S*)-Mn Chelate-5 than for the 1:1 mixture of *(S,S)-*Mn Chelate-5 and (*R,R*)-Mn Chelate-5 or Mn Chelate-5 synthesized without stereochemical control in brain and bone which is consistent with less Mn release in vivo. This is consistent with the Zn transmetallation data, and further supports the conclusion that the strength of Mn chelation as a function of chelate stereochemistry. This is further supported by higher level of Mn detected in the kidney for (*R*,*S*)-Mn Chelate-5, as more intact Mn Chelate-5 will pass through the kidney.

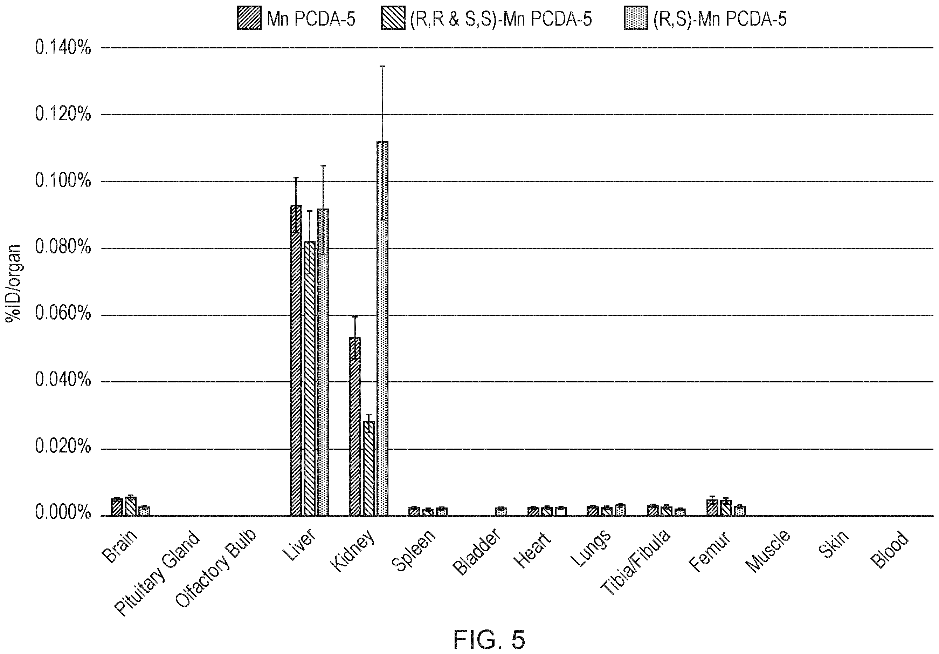

Since Mn Chelate-5 contains 10 additional stereocenters in the glucamine side-chain in addition to the 2 stereocenters alpha to the macrocycle, Mn Chelate-10 (Fig. 5) was synthesized without stereochemical control using racemic 3-amino-1,2-propanediol, and C¹⁸ chromatography afforded Mn Chelate-10 isomer pool A and Mn Chelate-10a isomer B. Zn transmetallation was performed with Mn Chelate-10 isomer pool A and Mn Chelate- 10a isomer B using the conditions described above (Fig. 9). Surprisingly, Mn Chelate-10 isomer pool A, (*S*,*S*)-Mn Chelate-5, and (*R,R*)-Mn Chelate-5 transmetallated with Zn and the same rate, and Mn Chelate-10 isomer pool B and *(R,S)* Mn Chelate-5 transmetallated with Zn and the same rate. This data shows that both the identity of the side-arm and the stereochemistry of the side-arm do not affect the strength of Mn chelation while the stereochemistry at the site alpha to the macrocycle is key in determining Mn chelate stability. Based on the data presented above, it is reasonable to assume that (R,S) and/or (*S,R)* stereochemistry alpha to the macrocycle is preferable with regard to Mn chelate stability as compared to *(R,R)* and (*S*,*S*) stereochemistry alpha to the macrocycle.

The following examples Ex. 1-8 describe preparation of precursors to the present invention. Examples 9-23 describe compounds according to various embodiments of the present invention.

### EXAMPLE 1

### Synthesis of N,N'-((methylazanediyl)bis(ethane-2,1-diyl))bis(4-methylbenzenesulfonamide) (Compound I).

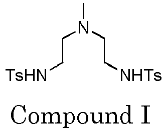

A 1 L round bottomed flask fitted with a magnetic stir bar was charged with N-tosylaziridine (49 g, 248 mmol) and AcN (450 mL). 41% aqueous methylamine (12 mL, 121 mmol) was added and stirred at ambient temperature for 36 h. A second aliquot of N-tosylaziridine (1.7 g, 8.62 mmol) was added and stirred at ambient temperature for an additional 48 h. The solvent was removed in vacuo and the crude residue was recrystallized from EtOH to give 45 g (87%) of the desired product as a white solid. 1H NMR (400 MHz, DMSO-D6, δ) 7.68 (4H, m), 7.36 (6H, m), 2.75 (4H, t), 2.38 (6H, s), 2.22 (4H, t), 1.93 (3H, s).

### EXAMPLE 2

### Synthesis of protected cyclic 2-arm chelate (Compound II).

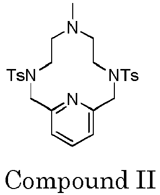

A 12L 3-necked round bottomed flask fitted with a reflux condenser and a mechanical stirrer was charged with N,N'-((methylazanediyl)bis(ethane-2,1-diyl))bis(4-methylbenzenesulfonamide (93 g, 218.5 mmol) and AcN (8.3 L). 2,6-bis(chloromethyl)pyridine (38.5 g, 218.5 mmol) was added and the resulting solution was heated at 80 ° for 16 h. The reaction mixture was cooled to ambient temperature and solvent was removed in vacuo until crystallization began. The resulting crystals were collected via vacuum filtration to afford 86.9 g (75%) of the desired product as a white solid (ESI: m/z = 530 (M + H)⁺).

### EXAMPLE 3

### Synthesis of deprotected 2-arm cyclic chelate (Compound III).

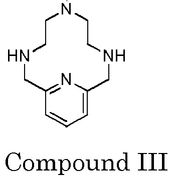

A 1 L 3-necked round bottomed flask fitted with a mechanical stirrer was charged with protected cyclic 2-arm chelate (150 g, 284 mmol) and concentrated sulfuric acid (250 mL, 4.69 mol) and heated at 100 °C for 15 h. The solution was poured onto ice and the pH was adjusted to 7.4 with the addition of 50 wt% NaOH in water resulting in the formation of a white solid. AcN (200 mL) was added and the white solid was removed via vacuum filtration. The filtrate was evaporated to dryness to give a brown foam. The foam was dissolved in water (200 mL) and purified with Amberlite A26 resin in its hydroxide form to give 61 g (98%) of the desired product as a tan solid. 1H NMR (400 MHz, AcN-D3, δ) 7.56 (1H, m), 7.03 (2H, m), 3.76 (4H, s), 2.47 (4H, m), 2.19 (3H, s), 1.95 (4H, s).

### EXAMPLE 4

### Synthesis of protected Mn 2-arm C5 chelate (Compound IV).

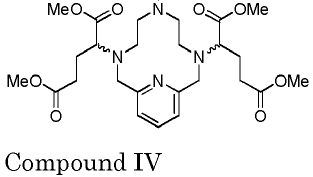

A 500 mL round bottomed flask fitted with a magnetic stir bar was charged with deprotected 2-arm cyclic chelate (20.0 g, 90.8 mmol) and AcN (160 mL). Diisopropylethylamine (38.7 mL, 217 mmol) and dimethyl 2-bromopentanedioate (47.7 g, 199.7 mmol) were added and the resulting solution was stirred at 65 °C for 20 h. Diisopropylethylamine (9.75 mL, 54.6 mmol) and dimethyl 2-bromopentanedioate (11.8 g, 49.4 mmol) were added and the resulting solution was stirred at 65 °C for an additional 19h. The solvent was removed in vacuo to leave a red oil. The oil was then dissolved in water (300 mL) and washed with EtOAc (300 mL). The EtOAc layer was then extracted with water (2 x 50 mL) and combined with the initial aqueous layer and the water was removed in vacuo to leave a red oil was used without further purification.

### EXAMPLE 5

### Synthesis of protected (R,R)-Mn 2-arm C5 chelate (Compound V).

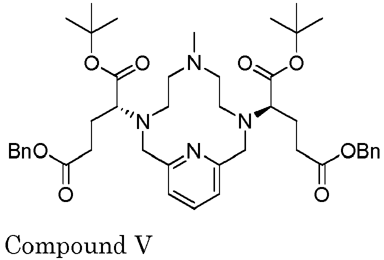

A 50 mL round bottomed flask fitted with a magnetic stir bar was charged with deprotected 2-arm cyclic chelate (1.01 g, 4.58 mmol), potassium carbonate (1.58 g, 11.5 mmol) and AcN (10 mL). (S)-5-benzyl 1-tert-butyl 2-(methylsulfonyloxy) pentanedioate (Levy, S.G. et al Org. Proc.Res. Dev. 2009, 13, 535-542) (2.60 g, 6.98 mmol) was dissolved in AcN (2 mL), then added to the stirred suspension. This mixture was heated in an oil bath at 50 °C for 21 h. An additional amount of (*S*)-5-benzyl 1-*tert*-butyl 2-(methylsulfonyloxy) pentanedioate (1.71 g, 4.59 mmol, in 2 mL of AcN) and potassium carbonate (317 mg, 2.29 mmol) were added and heating was continued for an additional 47 h. The reaction was then cooled to room temperature and filtered through a 0.45 µm PTFE filter. The solids were rinsed with acetonitrile (5 x 5 mL) and the combined filtrates were concentrated under reduced pressure which provided a red oil that was carried on without further purification (ESI: m/z = 774 (M + H)⁺). Specific rotation:[α]²⁷_{D}= +12.1° (c=0.00404, acetonitrile); ¹H NMR (CDCl₃, 500 MHz) δ 1.40-1.45 (m, 10H), 1.49 (s, 9H), 1.55-1.64 (m, 1H), 1.65-1.73 (m, 1H), 1.85-1.95 (m, 2H), 1.95-2.04 (m, 1H), 2.04-2.10 (m, 2H), 2.83-2.87 (m, 3H), 2.93-2.98 (m, 1H), 3.05-3.28 (m, 7H), 3.57 (d, *J* = 17.4 Hz, 1H), 3.70 (d, *J* = 16.9 Hz, 1H), 4.00 (d, *J* = 16.9 Hz, 1H), 4.03-4.14 (m, 2H), 4.25 (d, *J* = 17.6 Hz, 1H), 4.84-4.98 (m, 4H), 6.93 (d, *J* = 7.6 Hz, 1H), 6.99 (d, *J* = 7.6 Hz, 1H), 7.15-7.20 (m, 2H), 7.20-7.23 (m, 2H), 7.28-7.34 (m, 6H), 7.49 (t, *J* = 7.6 Hz, 1H), 11.45 (bs, 1H); ¹³C NMR (CDCl₃, 125 MHz) δ 25.5, 26.6, 28.3, 28.4, 30.4, 30.6, 33.9, 46.7, 52.9, 53.6, 54.3, 54.7, 57.7, 66.3, 66.5, 66.8, 67.9, 82.5, 82.6, 120.2, 120.6, 126.0, 128.5, 128.6, 128.7, 135.7, 135.8, 138.5, 159.8, 160.9, 170.7, 171.3, 172.1, 172.2.

### EXAMPLE 6

### Synthesis of protected (S,S)-Mn 2-arm C5 chelate (Compound VI).

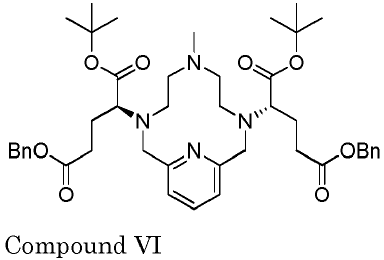

A 10 mL round bottomed flask fitted with a magnetic stir bar was charged with deprotected 2-arm cyclic chelate (0.210 g, 0.953 mmol), potassium carbonate (0.329 g, 2.38 mmol) and AcN (6 mL). (R)-5-benzyl 1-tert-butyl 2-(methylsulfonyloxy) pentanedioate (prepared from D-glutamic acid: Levy, S.G. et al Org. Proc.Res. Dev. 2009, 13, 535-542) (0.888 g, 2.38 mmol) was dissolved in AcN (2 mL), then added to the stirred suspension. This mixture was heated in an oil bath at 50 °C for 67 h. The reaction was then cooled to room temperature and concentrated under reduced pressure. The residue was partitioned between water and dichloromethane (20 mL each). The layers were separated and the aqueous later was extracted with dichloromethane (2 x 10 mL). The combined organics were back extracted with water (2 x 20 mL), then dried over anhydrous sodium sulfate. The dried organic layer was then filtered through a 0.45 µm PTFE filter and the filtrate was concentrated under reduced pressure which provided a yellow oil that was carried on without further purification (ESI: m/z = 774 (M + H)⁺).

### EXAMPLE 7

### Synthesis of protected (R,S)-Mn 2-arm C5 chelate (Compound VII).

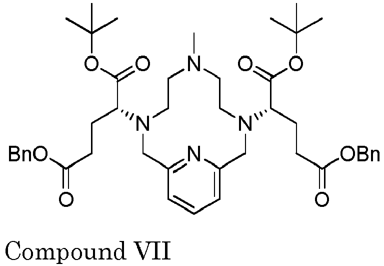

A 10 mL round bottomed flask fitted with a magnetic stir bar and nitrogen inlet was charged with deprotected 2-arm cyclic chelate (278 mg, 1.26 mmol) and isopropanol (4 mL). Diisopropylethylamine (522 µL, 3.07 mmol) was added followed by (S)-5-benzyl 1-tert-butyl 2-(methylsulfonyloxy) pentanedioate (prepared from L-glutamic acid: Levy, S.G. et al Org. Proc.Res. Dev. 2009, 13, 535-542) (407 mg, 0.87 mmol) and additional isopropanol (1 mL). The resulting solution was placed under nitrogen and heated in an oil bath at 50 °C for 48 h. An additional amount of (S)-5-benzyl 1-tert-butyl 2-(methylsulfonyloxy) pentanedioate (222 mg, 0.596 mmol) and diisopropylethylamine (215 µL, 1.26 mmol) were added and heating was continued for an additional 21 h. The reaction was cooled to room temperature and concentrated under reduced pressure. The residue was diluted with 15 mL of dichloromethane and 30 mL of water. The layers were separated, and the aqueous layer was extracted with 15 mL of dichloromethane. The combined organics were washed with 30 mL of water, then dried over sodium sulfate. The dried organics were filtered through a #1 filter and the resulting filtrate was concentrated under reduced pressure. The residual material was purified on C₁₈ column using a gradient elution (AcN/2 mM HCl, 10:90 to 80:20) to give 260 mg (43%) of the (R)-monoalkylated intermediate (ESI: m/z = 497 (M + H)⁺). A 10 mL round bottomed flask fitted with a magnetic stir bar and nitrogen inlet was charged with monoalkylated intermediate (260 mg, 0.524 mmol) and acetonitrile (5 mL). Potassium carbonate (219 mg, 1.58 mmol) was added followed by (R)-5-benzyl 1-tert-butyl 2-(methylsulfonyloxy) pentanedioate (prepared from D-glutamic acid: Levy, S.G. et al Org. Proc.Res. Dev. 2009, 13, 535-542) (252 mg, 0.676 mmol). The resulting solution was placed under nitrogen and heated in an oil bath at 50 °C for 48 h. An additional amount of (*R*)-5-benzyl 1-tert-butyl 2-(methylsulfonyloxy) pentanedioate (97 mg, 0.260 mmol) was added and heating was continued for an additional 48 hr. The reaction was cooled to room temperature, diluted with acetonitrile and filtered through a 0.45 µm filter. The yellow filtrate was concentrated under reduced pressure. The residue was dissolved in 1:1 acetonitrile:water (4 mL) and purified on C₁₈ column using a gradient elution (AcN/2 mM HCl, 10:90 to 80:20) to give 310 mg (76%, 32% over two steps) of the desired product (ESI: m/z = 774 (M + H)⁺); Specific rotation: [α]²⁴_{D =} +1.9° (c=0.044, acetonitrile); ¹H NMR (CDCl₃, 500 MHz) δ 1.38 (s, 18H), 1.37-1.45 (m, 2H), 1.69-1.80 (m, 4H), 1.80-1.89 (m, 2H), 2.63 (bs, 3H), 2.89-3.02 (m, 4H), 3.03-3.08 (m, 2H), 3.08-3.14 (m, 2H), 3.47 (d, *J* = 17.5 Hz, 2H), 3.96 (bs, 2H), 4.15 (d, *J* = 17.5 Hz, 2H), 4.79 (s, 4H), 6.90 (d, *J* = 7.5 Hz, 2H), 7.03-7.08 (m, 4H), 7.14-7.19 (m, 6H), 7.41 (t, *J* = 7.5 Hz, 1H), 11.83 (bs, 1H); ¹³C NMR (CDCl₃, 125 MHz) δ 26.1, 27.8, 28.0, 30.0, 52.1, 52.7, 53,4, 66.0, 67.4, 82.0, 119.8, 128.2, 128.3, 135.3, 138.6, 160.4, 170.9, 171.8.

### EXAMPLE 8

### Synthesis of deprotected (R,R)-Mn 2-arm C5 chelate (Compound VIII).

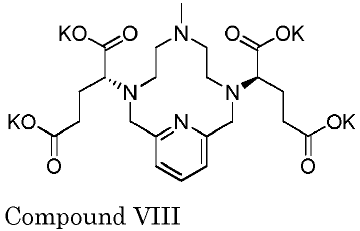

A 100 mL round bottomed flask fitted with a magnetic stir bar, reflux condenser and nitrogen inlet was charged with protected (R,R)-Mn 2-arm C5 chelate (3.64 g, 4.71 mmol) and dioxane (20 mL). 1 M HCl (20 mL, 20.0 mmol) was added and the mixture was placed under a nitrogen atmosphere and heated to 50 °C. After 5 days, a concentrated HCl solution was added (1.7 mL, 20.4 mmol) and heating was continued. After 3 days the reaction was cooled to room temperature and the solution was extracted with ethyl acetate (2 x 25 mL). The pH of the resulting aqueous layer was adjusted to ~8 with KOH(s) and this solution was concentrated under reduced pressure. Methanol (25 mL) was added to the residue and this mixture was stirred. The resulting slurry was filtered through a 0.45 µm PTFE filter. The filtrate was concentrated under reduced pressure to provide a brown foam that was carried on without further purification.

### EXAMPLE 9

### Synthesis of deprotected (S,S)-Mn-2-arm C5 chelate (Compound IX).

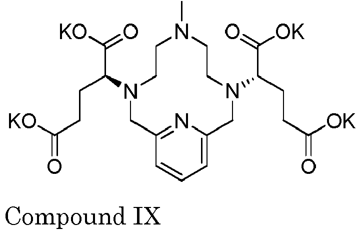

A 100 mL round bottomed flask fitted with a magnetic stir bar, reflux condenser and nitrogen inlet was charged with protected (*S*,*S*)-Mn 2-arm C5 chelate (0.713 g, 0.922 mmol) and dioxane (5 mL). Water (3.75 mL) was added followed by concentrated HCl (1.25 mL, 15.0 mmol) was added and the mixture was placed under a nitrogen atmosphere and heated to 50 °C. After 30 hr, the reaction was cooled to room temperature and the solution was extracted with ethyl acetate (2 x 10 mL). The combined organic extracts were back extracted with water (10 mL). The pH of the resulting aqueous layer was adjusted to ~8 with KOH pellets and this solution was concentrated under reduced pressure. Methanol (25 mL) was added to the residue and this mixture was stirred. The resulting slurry was filtered through a 0.45 µm PTFE filter. The filtrate was concentrated under reduced pressure to provide the desired product that was carried on without further purification (ESI: m/z = 481 (M + H)⁺).

### EXAMPLE 10

### Synthesis of deprotected (R,S)-Mn 2-arm C5 chelate (Compound X).

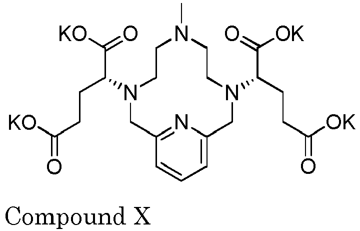

A 10 mL round bottomed flask fitted with a magnetic stir bar, reflux condenser and nitrogen inlet was charged with protected (*R*,*S*)-Mn 2-arm C5 chelate (189 mg, 0.245 mmol) and dioxane (2.5 mL). Water (1.25 mL) was added followed by concentrated HCl (1.25 mL) and the mixture was placed under a nitrogen atmosphere and heated to 50 °C for 40 h. The reaction was cooled to room temperature and the solution was diluted with water (15 mL) and ethyl acetate (10 mL). The layers were separated, and the aqueous layer was extracted with ethyl acetate (2 x 10 mL). The pH of the resulting aqueous layer was adjusted to ~7 with KOH(s) and this solution was concentrated under reduced pressure. Methanol (25 mL) was added to the residue and this mixture was stirred. The resulting slurry was filtered through a 0.45 µm PTFE filter. The filtrate was concentrated under reduced pressure providing the deprotected chelate that was carried on without further purification (ESI: m/z = 481 (M + H)⁺).

### EXAMPLE 11

### Synthesis of Mn 2-arm C5 chelate (Compound XI).

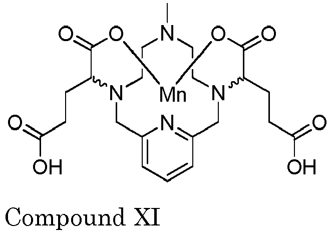

A 1L round bottomed flask fitted with a magnetic stir bar was charged with protected Mn 2-arm C5 chelate (48.7 g, 90.8 mmol) and water (450 mL). Sodium hydroxide (29.1 g, 726 mmol) was added and stirred at ambient temperature for 2 h. The reaction mixture was washed with EtOAc (250 mL) and the layers were separated. The aqueous layer was washed again with EtOAc (2 x 100 mL) and the aqueous layer was collected. Manganese chloride tetrahydrate (19.6 g, 99 mmol) was added to the aqueous solution. The pH was adjusted to 7.1 with 6M NaOH and stirred at ambient temperature for 17 h and then at 90 °C for 2.5 h. After cooling to ambient temperature, the pH was adjusted to 10.1 with 50 wt% aqueous NaOH and a fine brown precipitate formed. The precipitate was removed via centrifugation at 3000 rcf for 20 min and the supernatant was collected and evaporated to dryness in vacuo. The residue was triturated with MeOH (127 mL) at 40 °C for 1.5 h. The insoluble white solid was removed via centrifugation at 3000 rcf for 30 min. The supernatant was evaporated to dryness in vacuo to give an off-white solid which was purified on C18 silica gel (3% AcN in water) to give 36.8 g (75%) of the desired product as an off white solid (ESI: m/z = 534 (M + H)⁺).

### EXAMPLE 12

### Synthesis of (R,R)-Mn 2-arm C5 chelate (Compound XII).

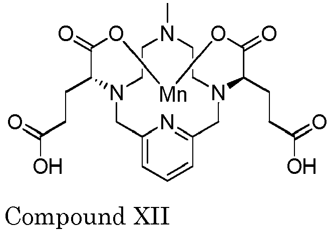

A 25 mL round bottomed flask fitted with a magnetic stir bar was charged with deprotected (*R,R)*-Mn 2-arm C5 chelate (2.98 g, 4.71 mmol) and water (12 mL). Manganese chloride tetrahydrate (1.41 g, 7.12 mmol) was added to the aqueous solution. The pH was adjusted to 6.5 with 50% aqueous KOH and stirred at ambient temperature for 5 h. The pH was adjusted to 10 with KOH(s) and the mixture was stirred overnight. The brown suspension was filtered through a 0.45 µm PTFE filter containing celite (4.9 cm2 x 1.8 cm). The pH of the filtrate was adjusted to 7.3 with cHCl (20 µL) and this material was concentrated under reduced pressure. The residual material was combined with methanol (25 mL) and stirred. The resulting slurry was filtered through a 0.45 µm PTFE filter and the solid was rinsed several times with methanol. The filtrate was concentrated under reduced pressure and the residual material was purified on C¹⁸ column (AcN/water) to give 1.09 g (43%) of the desired product (ESI: m/z = 534 (M + H)⁺).

### EXAMPLE 13

### Synthesis of (S,S)-Mn 2-arm C5 chelate (Compound XIII).

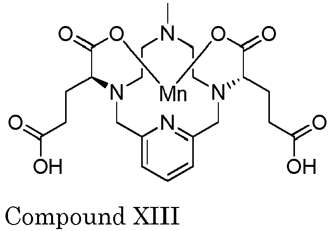

In a procedure identical to its diastereomer, (*S*,*S*)-Mn 2-arm C5 chelate (0.092 g, 0.17 mmol) produced 0.101 g (68%) of the expected product (ESI: m/z = 860 (M + H)⁺). Specific rotation:[α]²⁴_{D =}-3.0° (c=0.018, water).

### EXAMPLE 14

### Synthesis of (R,S)-Mn 2-arm C5 chelate (Compound XIV).

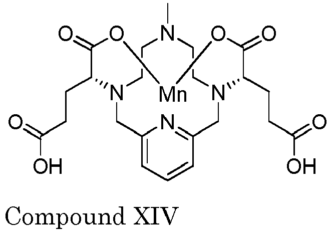

A 5 mL round bottomed flask fitted with a magnetic stir bar was charged with deprotected (*R*,*S*)-Mn 2-arm C5 chelate (180 mg, 0.284 mmol) and water (4 mL). Manganese chloride tetrahydrate (115 mg, 0.581 mmol) was added to the aqueous solution. The pH of the solution was adjusted from 6.0 to 6.4 with 50% aqueous KOH and stirred at ambient temperature for 4 d. The pH was adjusted to 10 with KOH(s) and the mixture was stirred overnight. The brown suspension was filtered through a 0.45 µm PTFE filter containing celite (4.9 cm² x 1.8 cm). The pH of the filtrate was adjusted to 7.2 with cHCl (8 µL) and this material was concentrated under reduced pressure. The residual material was combined with methanol (25 mL) and stirred. The resulting slurry was filtered through a 0.45 µm PTFE filter and the solid was rinsed several times with methanol. The filtrate was concentrated under reduced pressure and the residual material was purified on C₁₈ column, eluting with water to give 94 g (62%) of the desired product (ESI: m/z = 534 (M + H)⁺).

### EXAMPLE 15

### Synthesis of Mn Chelate-5 (Compound XV).

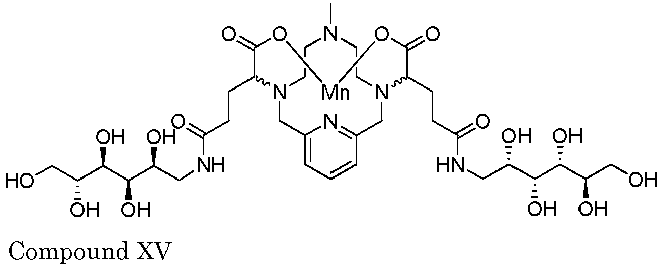

A 50 mL 2-necked flask fitted with a magnetic stir bar was charged with D-glucamine (0.713 g, 3.94 mmol) and water (19.7 mL). The pH of the resulting solution was adjusted to 7.4 with 1.0 M HCl and Mn 2-arm C5 chelate (1.00 g, 1.87 mmol) was added followed by EDCI-HCl (0.848 g, 4.42 mmol) and HOBt hydrate (0.121 g, 0.787 mmol). The pH was maintained at 6 with addition of 1.0 M HCl or 1.0 M NaOH as needed while stirring at ambient temperature for 8 h. D-Glucamine (0.359 g, 1.98 mmol) and EDCI-HCl (0.433 g, 2.26 mmol)) were added and the pH was maintained at 6 while stirring at ambient temperature for 16 h. The reaction solution was evaporated to dryness in vacuo and the crude product was purified on C18 silica gel (100% water to 20% AcN in water) to give 0.782 g (48 %) of the desired product as a pale-yellow solid (ESI: m/z = 860 (M + H)⁺).

### EXAMPLE 16

### Synthesis of (R,R)-Mn Chelate-5 (Compound XVI).

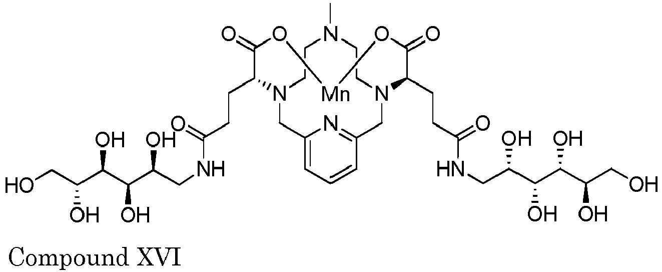

A 25 mL flask fitted with a magnetic stir bar was charged with (*R,R)-*Mn 2-arm C5 chelate (1.06 g, 2.04 mmol), D-glucamine (0.833 g, 4.60 mmol), HOBt hydrate (0.016 g, 0.10 mmol) and water (8.2 mL). The pH of the resulting solution was adjusted to 6.2 with concentrated HCl. EDCI-HCl (0.924 g, 4.82 mmol) was added and the pH was maintained between 6-6.5 with addition of concentrated HCl as needed while stirring at ambient temperature for 23 h. The reaction solution was diluted with water (30 mL) and purified by sequentially passing the solution through an IR-120(Na) ion exchange column followed by an IR-400(Cl) ion exchange column. The crude product was purified on C¹⁸ silica gel (2% AcN in water to 50% AcN in water) to give 1.42 g (81 %) of the desired product as a pale-yellow foam (ESI: m/z = 860 (M + H)⁺). Specific rotation: [α]26D = -19.7° (c=0.0129, water).

### EXAMPLE 17

### Synthesis of (S,S)-Mn Chelate-5 (Compound XVII).

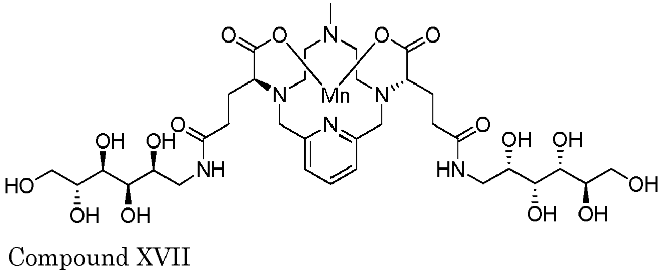

In a procedure identical to its diastereomer, (*S*,*S*)-Mn 2-arm C5 chelate (0.092 g, 0.17 mmol) produced 0.101 g (68%) of the expected product (ESI: m/z = 860 (M + H)⁺). Specific rotation: [α]24D = -3.0° (c=0.018, water).

### EXAMPLE 18

### Synthesis of (R,S)-Mn Chelate-5 (Compound XVIII).

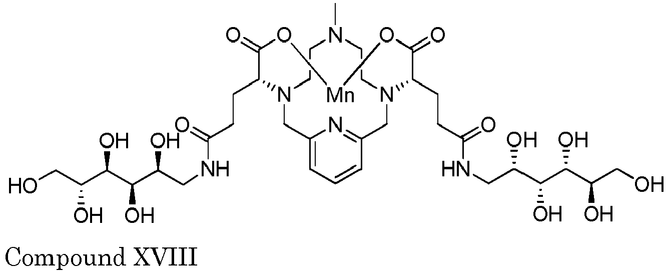

A 5 mL flask fitted with a magnetic stir bar was charged with *(R,S)-*Mn Chelate-1a (60 mg, 0.112 mmol), D-glucamine (50 mg, 0.276 mmol), HOBt hydrate (4.6 mg, 0.030 mmol) and water (3 mL). The pH of the resulting solution was adjusted to 6 with concentrated HCl (40 µL). EDCI-HCl (52 mg, 0.271 mmol) was added and the mixture was stirred at ambient temperature. Additional amounts of EDCI were added after 6 h (44 mg, 0.230 mmol), 24 h (40 mg, 0.209 mmol) and 30 h (50 mg, 0.261 mmol). The reaction solution was diluted with water (3 mL) and purified by sequentially passing the solution through an IR-120(Na) ion exchange column followed by an IR-400(Cl) ion exchange column. The crude product was purified on a C₁₈ column using a gradient elution (AcN/water, 0:100 to 10:90) to give 23 mg of (*S*,*R*)-Mn Chelate-5 (24%, 42% d.e.) and 40 mg of (*R,S*)-Mn Chelate-5 (42%, 91% d.e.) (ESI: m/z = 860 (M + H)⁺).

### EXAMPLE 19

Isomerization of (*S*,*R*)-Mn 2-arm C5 chelate to (*R*,*S*)-Mn 2-arm C5.

A mixture of (*R,S*)-Mn Chelate- 1a and (*S,R*)-Mn Chelate- 1a (59 mg, 0.111 mmol) was dissolved in water (1 mL). This mixture was sealed in a glass vial and heated to 90 °C for 42 h. After this heating period, the HPLC showed a collapse of the two peaks into one peak. (ESI: m/z = 534 (M + H)⁺).

### EXAMPLE 20

Synthesis of (*R*,*S*)-Mn Chelate-5 prepared from isomerized (*S*,*R*)-Mn 2-arm C5 chelate.

To a HPLC vial containing (*R,S*)-Mn Chelate- 1a (59 mg, 0.111 mmol) and water (1 mL) was added D-glucamine (49 mg, 0.270 mmol) and HOBt hydrate (5 mg, 0.032 mmol). The pH of the resulting solution was adjusted to 5.7 with concentrated HCl (35 µL). EDCI-HCl (53 mg, 0.276 mmol) was added and the mixture was stirred at ambient temperature for 22 h. More EDCI was added (23 mg, 0.120 mmol) and the reaction was stirred for an additional 42 h. The reaction solution was diluted with water (3 mL) and purified by sequentially passing the solution through an IR-120(Na) ion exchange column followed by an IR-400(Cl) ion exchange column. The crude product was purified on a C₁₈ column using a gradient elution (AcN/water, 0:100 to 10:90) to give 12 mg (37%, 74% d.e.) of the desired (R,S) isomer as an off-white solid (ESI: m/z = 860 (M + H)⁺).

### EXAMPLE 21

### Synthesis of Mn Chelate-10 (Compound XIX).

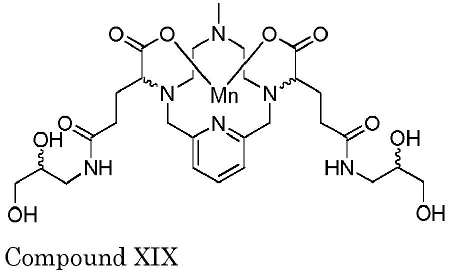

*rac*-3-aminopropane-1,2-diol (0.190 g, 2.08 mmol) was dissolved in H₂O (10.4 mL) in a 25 mL 2-necked round bottomed flask fitted with magnetic stir bar and a pH probe. The resulting solution was adjusted to pH 7.1 with 1.0 M HCl and Mn Chelate-1a (0.603 g, 0.996 mmol) was added followed by EDCI-HCl (0.473 g, 2.47 mmol) and then HOBt hydrate (0.063 g, 0.466 mmol). The pH was maintained at 6 with addition of 1.0 M HCl or 1.0 M NaOH as needed while stirring at ambient temperature for 7.5 h. rac-3-aminopropane-1,2-diol (0.095 g, 1.04 mmol) and EDCI-HCl (0.453 g, 2.36 mmol) were added and the pH was maintained at 6 while stirring at ambient temperature for 15 h. The reaction solution was evaporated to dryness in vacuo and the crude product was purified on C¹⁸ silica gel (100% water to 20% AcN in water) to give 0.280 g (41 %) of the desired product as a pale-yellow solid (ESI: m/z = 680 (M + H)⁺).

### EXAMPLE 22

Synthesis of Mn Chelate-10 isomer pool A and isomer pool B. Mn Chelate-10 was synthesized as described above and the 2 isomer pools were separated and isolated on C¹⁸ silica gel (100% water to 20% AcN in water) as pale-yellow solids (ESI: m/z = 680 (M + H)⁺).

### EXAMPLE 23

### Synthesis of Mn Chelate-15 (Compound XX).

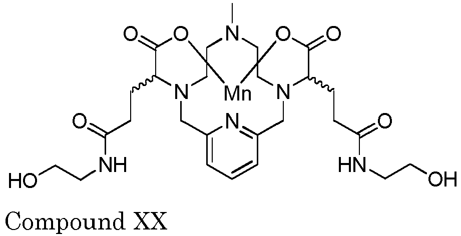

Ethanolamine (5.153 g, 84.4 mmol) was dissolved in H₂O (50 mL) in a 250 mL 3-necked round bottomed flask fitted with magnetic stir bar and a pH probe. Mn Chelate-1a (20.107 g, 37.7 mmol) was added followed by HOBt hydrate (0.289 g, 1.89 mmol). The pH was adjusted to 6.3 with conc. HCl and EDCI-HCl (16.966 g, 88.5 mmol) was then added. The pH was maintained between 6.0 and 6.5 while stirring at ambient temperature for 2.5 h. The reaction solution was diluted with water (280 mL) and purified by sequentially passing the solution through an IR-120(Na) ion exchange column followed by an IR-400(Cl) ion exchange column. The resulting crude material was then purified on C¹⁸ silica gel (5% AcN to 20% AcN in water) to give 16.05 g (69 %) of the desired product as a pale-yellow solid (ESI: m/z = 620 (M + H)⁺).

### EXAMPLE 24

Synthesis of Mn Chelate-15 isomer pool A and isomer pool B. Mn Chelate-15 was synthesized as described above and the 2 isomer pools were separated and isolated on C¹⁸ silica gel (5% AcN to 20% AcN in water) as pale-yellow solids (ESI: m/z = 620 (M + H)⁺).

### EXAMPLE 25

General method for measurement of r1 and r2 relaxivities. Manganese containing chelates were dissolved in water at concentrations ranging from 5 to 0 mM Mn. T1 and T2 relaxation times were then measured at 60 MHz and 40 °C using a Brüker mq60 relaxometer. Linear fits (R² > 0.99 in all cases) of 1/T1 or 1/T2 as a function of Mn concentration gave r1 or r2 values respectively.

**Table 1. r1 and r2 relaxivities in human serum at 60 MHz and 37 °C.**

| Compound | r1 (mM⁻¹s⁻¹) | r2 (mM⁻¹s⁻¹) |
|---|---|---|
| Mn Chelate-5 | 6.0 | 15.4 |
| 1:1 mixture of (R,R) & (S,S)-Mn Chelate-5 | 5.9 | 15.2 |
| (R,S)-Mn Chelate-5 | 6.0 | 15.4 |

Method for assessing trans-metalation with Zn. Mn chelates were dissolved in aqueous solutions containing 200 mM ZnCl₂ and 15 mM ammonium formate at either pH = 4 to give a Mn chelate concentration of approx. 2 mM. The resulting solutions were incubated at 40 °C with mixing and aliquots were periodically analyzed by HPLC. The percent Mn containing chelate remaining in the solution was measured by integration at 265 nm.

Method for preparation of Mn-54 labeled chelate for biodistribution studies. To a 3 mL glass vial fitted with a magnetic stir bar was added the manganese containing chelate (1 mg) and 1.0 M ammonium formate, pH = 4 (0.5 mL). ⁵⁴MnCl₂ in 1.0 M HCl (~500 µCi) was then added and the resulting solution was heated at 40 °C for 16 h. The resulting solution was purified via preparative HPLC to remove unchelated Mn. The radioactive fraction was collected and evaporated to dryness in vacuo. The radioactive residue was taken up in water containing non-radioactive Mn chelate (0.310 M) such that ~30 µCi of radioactivity was formulated in a dose of 0.620 mmol Mn/kg with an injection volume of 2 mL/kg.

General method for Mn-54 biodistribution studies. The experimental protocol conformed to the Guide for the Care and Use of Laboratory Animals and was approved by the institutional IACUC. Female Sprague-Dawley rats (130-150g) were housed in standard cages, provided with ad libitum access to standard commercial feed and water, and were maintained on an alternating 12-hr light:dark cycle in rooms with controlled temperature and humidity. Prior to injection of Mn-54 labeled chelates, rats were anesthetized via inhaled 3% Isofluorane (EZ Anesthesia Systems). The injection site was prepped with alcohol wipes and a temporary 27Ga catheter was placed in a tail vein. ~30 µCi of Mn-54 labeled chelate formulated with non-radioactive Mn chelate was dosed at 0.620 mmol non-radioactive Mn chelate/kg at an injection volume of 2 mL/kg was injected at a rate of 1mL/min. Following injection animals were individually housed in wire bottom cages with lined with filter paper until first urine void urine was collected. The rats were then cohoused in standard long term caging. 7 days post injection, the animals were sacrificed by CO2 immersion and organs and tissues of interest were removed and assayed for radioactivity using a Wizard 2480 gamma counter (Table 2).

**Table 2. %ID ± standard deviation for tissues 7d post injection of a 0.62 mmol Mn/kg dose containing ~ 30 µCi of ⁵⁴Mn labeled chelate.**

| **Tissue** | **Mn Chelate-5^{a}** | **1:1 mixture of (R,R) & (S,S)-Mn Chelate-5^{b}** | **(R,S)-Mn Chelate-5^{b}** |
|---|---|---|---|
| Brain | 0.005 ± 0.0004% | 0.005 ± 0.001% | 0.002 ± 0.0004% |
| Pituitary Gland | ≤ LOD | ≤ LOD | ≤ LOD |
| Olfactory Bulb | ≤ LOD | ≤ LOD | ≤ LOD |
| Liver | 0.093 ± 0.008% | 0.082 ± 0.009% | 0.091 ± 0.013% |
| Kidney | 0.053 ± 0.006% | 0.027 ± 0.003% | 0.112 ± 0.023% |
| Spleen | ≤ LOD | ≤ LOD | 0.002 ± 0.0002% |
| Bladder | ≤ LOD | ≤ LOD | 0.002 ± 0.0003% |
| Heart | ≤ LOD | 0.002 ± 0.0004% | 0.002 ± 0.0002% |
| Lungs | 0.003 ± 0.0004% | 0.003 ± 0.001% | 0.003 ± 0.0003% |
| Muscle | ≤ LOD | ≤ LOD | ≤ LOD |
| Skin | ≤ LOD | ≤ LOD | ≤ LOD |
| Blood | ≤ LOD | ≤ LOD | ≤ LOD |
| Femur | 0.004 ± 0.001% | 0.004 ± 0.0006% | 0.002 ± 0.0004% |

| | | | |
|---|---|---|---|
| ^{a}LoD = 0.002% ID, n = 4; ^{b}LoD = 0.001% ID, n = 4 | | | |

### EXAMPLE 26

The following method was used for measurement of dissociation of manganese from the manganese chelate in human serum.

To human serum obtained from clotted blood (Sigma-Aldrich, 1900 µL) was added an aqueous solution of the manganese chelate (2 mM, 100 µL). The mixture was incubated at 37 °C. At specific time points aliquots of 200 µL of test sample were mixed with saline (400 µL) and analysed by size exclusion chromatography (metal-free HPLC equipped with a column with separation range 5 000-500 000 Dalton) combined with online detection of manganese containing species by ICP-SF-MS. Percent dechelation was measured as area percent of the high molecular weight (protein) fraction. At regular intervals EDTA solution was run through the system to ensure low levels of background metal ions. Human serum was incubated with Mn(II)acetate and injected to identify the position of Mn-binding components in the high molecular weight region of the chromatogram.

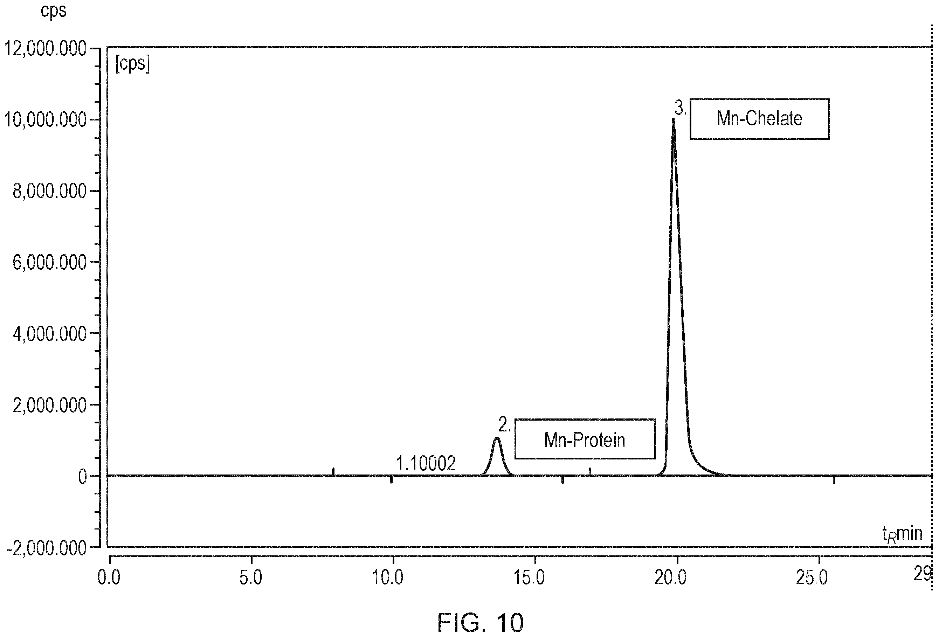

Fig. 10 illustrates the chromatographic separation of protein bound manganese from intact manganese chelate in the more slowly eluting low molecular weight fraction.

Four different stereoisomers of Mn Chelate-5 (RR, SS, SR and RS) were analysed in this assay. **Table 3** shows percent dechelation as measured by quantitative detection of protein bound manganese at time points 3, 24 and 49 h after the start of the experiment.

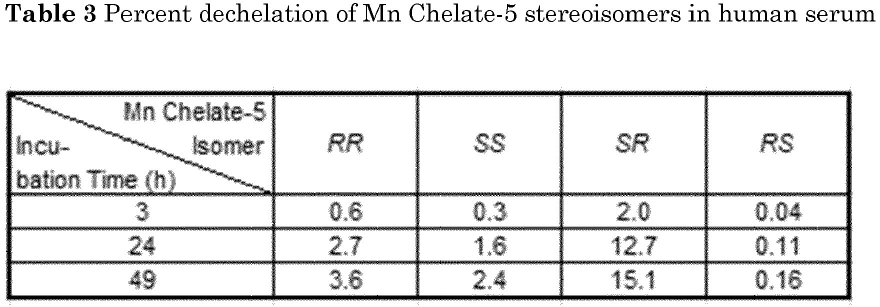

## Claims

1. A compound of formula IA: 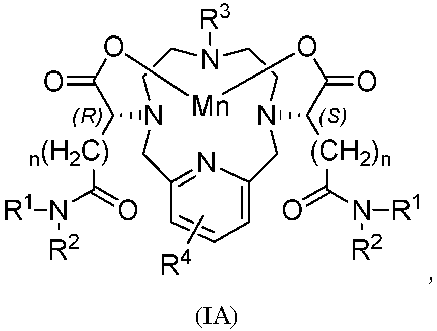 or a salt or solvate thereof, wherein:
each R¹ is independently selected from C₁₋₂₀ hydroxyalkyl, C₁₋₆ alkyl, C₃₋₆ aryl optionally-substituted with one or more substituents selected from halo and - C(=O)-NH-C₁₋₆ hydroxyalkyl, or a carbohydrate moiety;
each R² is independently selected from C₁₋₂₀ hydroxyalkyl, C₁₋₆ alkyl or hydrogen;
R³ is selected from C₁₋₃ alkyl or -(CH₂)ₘ-C(=O)- NR⁵R⁶, wherein m is an integer from 2-5, wherein R⁵ and R⁶ are independently selected from hydrogen, from C₁₋₂₀ hydroxyalkyl, C₁₋₆ alkyl, C₃₋₆ aryl optionally-substituted with one or more substituents selected from halo and -C(=O)-NH-C₁₋₆ hydroxyalkyl, or a carbohydrate moiety;
R⁴ represents 0-3 substituents selected from hydroxy, C₁₋₆ alkyl and C₁₋₆ hydroxyalkyl; and,
each n is an integer from 0-4.

2. The compound of claim 1, wherein the compound is of Formula IA,
each R¹ is C₁₋₂₀ hydroxyalkyl;
R³ is methyl;
R² and R⁴ are hydrogen; and
n is 2.

3. A compound having the structure:

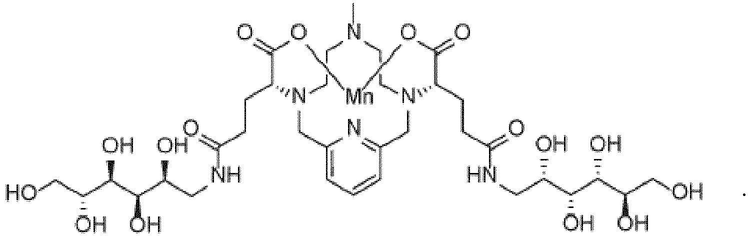

4. A composition comprising a compound of formula IA: 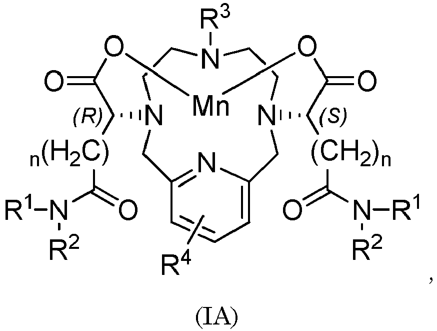 or a salt or solvate thereof, and a pharmaceutically acceptable excipient, wherein:
each R¹ is independently selected from C₁₋₂₀ hydroxyalkyl, C₁₋₆ alkyl, C₃₋₆ aryl optionally-substituted with one or more substituents selected from halo and - C(=O)-NH-C₁₋₆ hydroxyalkyl, or a carbohydrate moiety;
each R² is independently selected from C₁₋₂₀ hydroxyalkyl, C₁₋₆ alkyl or hydrogen;
R³ is selected from C₁₋₃ alkyl or -(CH₂)ₘ-C(=O)- NR⁵R⁶, wherein m is an integer from 2-5, wherein R⁵ and R⁶ are independently selected from hydrogen, from C₁₋₂₀ hydroxyalkyl, C₁₋₆ alkyl, C₃₋₆ aryl optionally-substituted with one or more substituents selected from halo and -C(=O)-NH-C₁₋₆ hydroxyalkyl, or a carbohydrate moiety;
R⁴ represents 0-3 substituents selected from hydroxy, C₁₋₆ alkyl and C₁₋₆ hydroxyalkyl; and,
each n is an integer from 0-4.

5. The compound of claim 1 or the composition of claim 4, wherein each R¹ is:
(i) C₁₋₂₀ hydroxyalkyl; or
(ii) independently C₃₋₉ hydroxyalkyl; or
(iii) independently C₃₋₆ hydroxyalkyl.

6. The compound of claim 1 or the composition of claim 4, wherein R³ is methyl.

7. The compound of claim 1 or the composition of claim 4, wherein R² and R⁴ are hydrogen.

8. The compound of claim 1 or the composition of claim 4, wherein n is 2.

9. The compound of claim 1 or the composition of claim 4, where the compound of Formula IA comprises at least two hydroxy groups.

10. The composition of claim 4, wherein the compound of Formula IA has the structure:

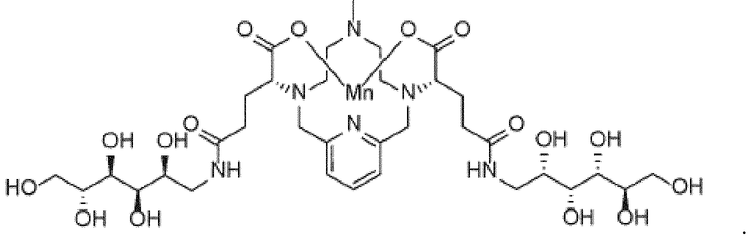

11. The composition of claim 4, wherein the composition lacks detectable amounts of the compound of Formula (IB): 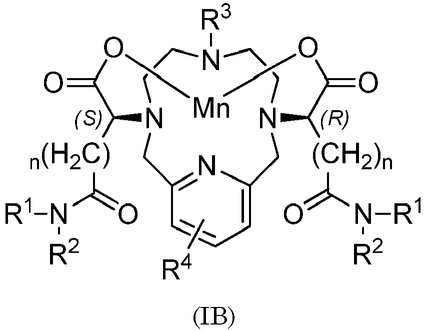 or a salt or solvate thereof.

12. The composition of claim 11, wherein the compound of Formula (IB) has the structure:

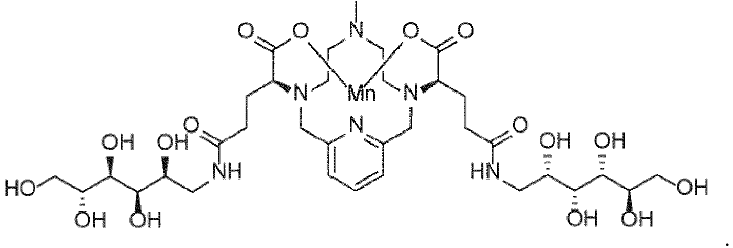

13. The composition of claim 4, wherein the composition further comprises Mn Chelates having (*R,R*) and (*S*,*S*) stereochemistry.

14. A method of imaging a patient comprising administering the compound of claim 1 or claim 3 to a patient in need thereof, followed by acquiring an MRI image of the patient.

15. A method of imaging a patient comprising administering the composition of claim 4 or claim 10 to a patient in need thereof, followed by acquiring an MRI image of the patient.

16. A method of enantioselective synthesis of the compound of Formula (IA) of claim 1 comprising:
(a) monoalkylation using a first enantiomer of 5-benzyl 1-tert-butyl 2-(methylsulfonyloxy) pentanedioate of a compound of formula (III) 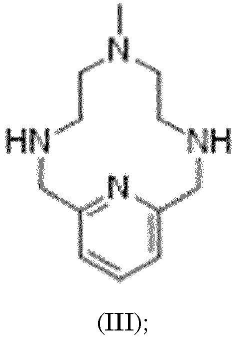
(b) followed by alkylation of the compound from step (a) with a second enantiomer of 5-benzyl 1-tert-butyl 2-(methylsulfonyloxy) pentanedioate, the second enantiomer being opposite the first enantiomer;
(c) reacting the product of step (b) with Mn; and
(d) reacting the product of step (c) with an aminoalcohol.

17. A method of enantioselective synthesis of a composition comprising Formula (IA) of claim 4 comprising:
(a) monoalkylation using a first enantiomer of 5-benzyl 1-tert-butyl 2-(methylsulfonyloxy) pentanedioate of a compound of formula (III) 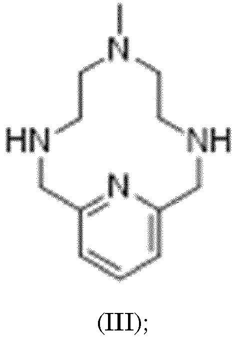
(b) followed by alkylation of the compound from step (a) with a second enantiomer of 5-benzyl 1-tert-butyl 2-(methylsulfonyloxy) pentanedioate, the second enantiomer being opposite the first enantiomer;
(c) reacting the product of step (b) with Mn; and
(d) reacting the product of step (c) with an aminoalcohol.

18. A method of making the composition of claim 4, wherein the method comprises:
heating a composition comprising the compound of Formula (IB): 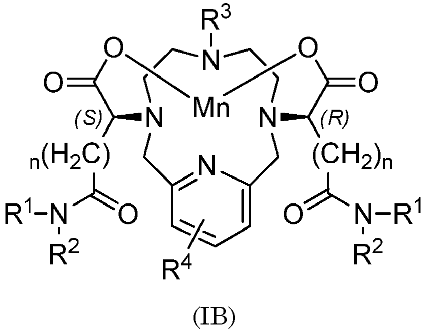 or a salt or solvate thereof;
wherein the heating is for a sufficient time and temperature to convert substantially all of the compound of Formula (IB) into the compound of Formula (IA), optionally
wherein the resulting composition lacks detectable amounts of the compound of Formula (IB).

## Patentansprüche

1. Verbindung der Formel IA: 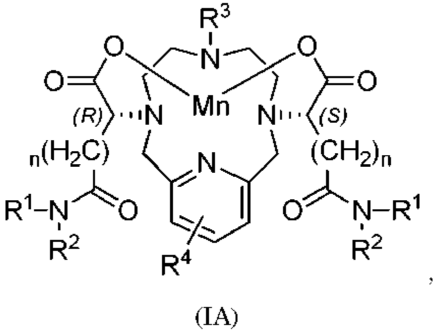 oder ein Salz oder Solvat davon, wobei:
R¹ jeweils unabhängig aus C₁₋₂₀-Hydroxyalkyl, C₁₋₆-Alkyl, C₃₋₆-Aryl, das gegebenenfalls durch einen oder mehrere Substituenten, die aus Halogen und -C (=O)-NH-C₁₋₆-Hydroxyalkyl ausgewählt sind, oder einer Kohlenhydratgruppierung ausgewählt ist;
R² jeweils unabhängig aus C₁₋₂₀-Hydroxyalkyl, C₁₋₆-Alkyl oder Wasserstoff ausgewählt ist;
R³ aus C₁₋₃-Alkyl oder - (CH₂)ₘ-C(=O)-NR⁵R⁶ ausgewählt ist, wobei m für eine ganze Zahl von 2-5 steht, wobei R⁵ und R⁶ unabhängig aus Wasserstoff, aus C₁₋₂₀-Hydroxyalkyl, C₁₋₆-Alkyl, C₃₋₆-Aryl, das gegebenenfalls durch einen oder mehrere Substituenten, die aus Halogen und -C(=O)-NH-C₁₋₆-Hydroxyalkyl ausgewählt sind, substituiert ist, oder einer Kohlenhydratgruppierung ausgewählt sind;
R⁴ 0-3 Substituenten repräsentiert, die aus Hydroxy, C₁-₆-Alkyl und C₁₋₆-Hydroxyalkyl ausgewählt sind; und
n jeweils für eine ganze Zahl von 0-4 steht.

2. Verbindung nach Anspruch 1, wobei die Verbindung die Formel IA aufweist,
R¹ jeweils für C₁₋₂₀-Hydroxyalkyl steht;
R³ für Methyl steht;
R² und R⁴ für Wasserstoff stehen; und
n für 2 steht.

3. Verbindung mit der Struktur:

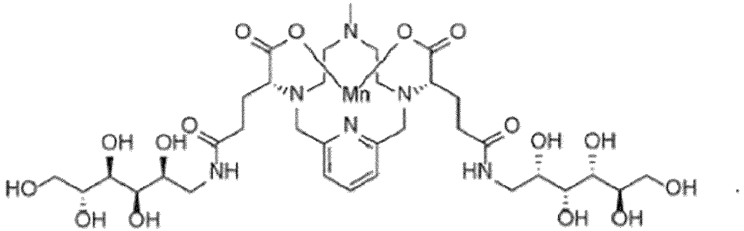

4. Zusammensetzung, umfassend eine Verbindung der Formel IA: 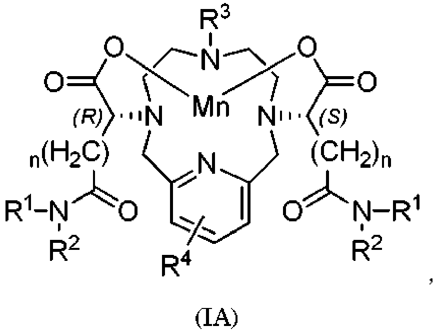 oder ein Salz oder Solvat davon und einen pharmazeutisch unbedenklichen Hilfsstoff, wobei:
R¹ jeweils unabhängig aus C₁₋₂₀-Hydroxyalkyl, C₁₋₆-Alkyl, C₃₋₆-Aryl, das gegebenenfalls durch einen oder mehrere Substituenten, die aus Halogen und -C (=O)-NH-C₁₋₆-Hydroxyalkyl ausgewählt sind, oder einer Kohlenhydratgruppierung ausgewählt ist;
R² jeweils unabhängig aus C₁₋₂₀-Hydroxyalkyl, C₁₋₆-Alkyl oder Wasserstoff ausgewählt ist;
R³ aus C₁₋₃-Alkyl oder - (CH₂)ₘ-C(=O)-NR⁵R⁶ ausgewählt ist, wobei m für eine ganze Zahl von 2-5 steht, wobei R⁵ und R⁶ unabhängig aus Wasserstoff, aus C₁₋₂₀-Hydroxyalkyl, C₁₋₆-Alkyl, C₃₋₆-Aryl, das gegebenenfalls durch einen oder mehrere Substituenten, die aus Halogen und -C(=O)-NH-C₁₋₆-Hydroxyalkyl ausgewählt sind, substituiert ist, oder einer Kohlenhydratgruppierung ausgewählt sind;
R⁴ 0-3 Substituenten repräsentiert, die aus Hydroxy, C₁₋₆-Alkyl und C₁₋₆-Hydroxyalkyl ausgewählt sind; und
n jeweils für eine ganze Zahl von 0-4 steht.

5. Verbindung nach Anspruch 1 oder Zusammensetzung nach Anspruch 4, wobei R¹ jeweils für Folgendes steht:
(i) C₁₋₂₀-Hydroxyalkyl; oder
(ii) unabhängig C₃₋₉-Hydroxyalkyl; oder
(iii) unabhängig C₃₋₆-Hydroxyalkyl.

6. Verbindung nach Anspruch 1 oder Zusammensetzung nach Anspruch 4, wobei R³ für Methyl steht.

7. Verbindung nach Anspruch 1 oder Zusammensetzung nach Anspruch 4, wobei R² und R⁴ für Wasserstoff stehen.

8. Verbindung nach Anspruch 1 oder Zusammensetzung nach Anspruch 4, wobei n für 2 steht.

9. Verbindung nach Anspruch 1 oder Zusammensetzung nach Anspruch 4, wobei die Verbindung der Formel IA mindestens zwei Hydroxylgruppen umfasst.

10. Zusammensetzung nach Anspruch 4, wobei die Verbindung der Formel IA die folgende Struktur aufweist:

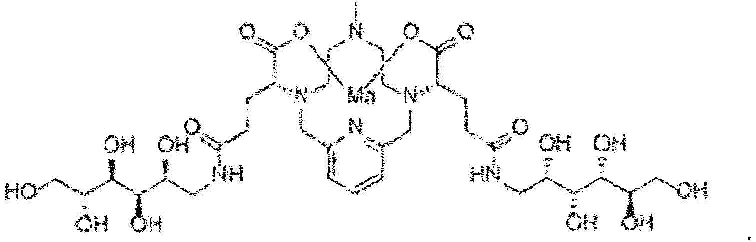

11. Zusammensetzung nach Anspruch 4, wobei die Zusammensetzung keine nachweisbaren Mengen der Verbindung der Formel (IB): 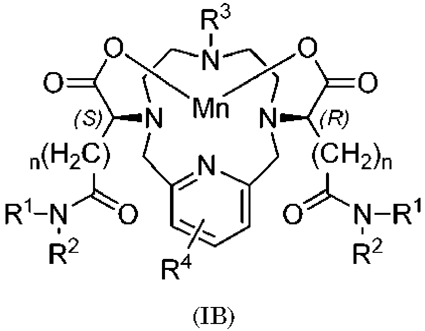 oder eines Salzes oder Solvats davon enthält.

12. Zusammensetzung nach Anspruch 11, wobei die Verbindung der Formel (IB) die folgende Struktur aufweist:

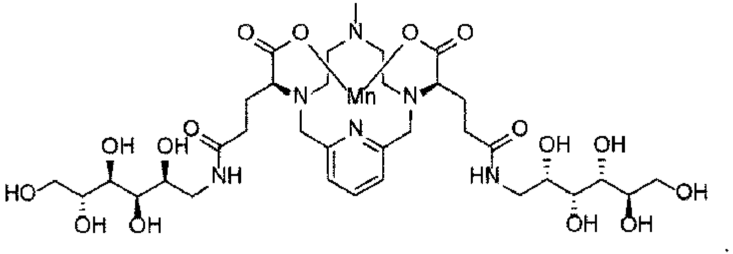

13. Zusammensetzung nach Anspruch 4, wobei die Zusammensetzung ferner Mn-Chelate mit (R,R)- und (S,S)-Stereochemie umfasst.

14. Verfahren zum Abbilden eines Patienten, umfassend das Verabreichen der Verbindung nach Anspruch 1 oder Anspruch 3 an einen Patienten, bei dem diesbezüglicher Bedarf besteht, gefolgt von der Aufnahme eines MRI-Bilds des Patienten.

15. Verfahren zum Abbilden eines Patienten, umfassend das Verabreichen der Zusammensetzung nach Anspruch 4 oder Anspruch 10 an einen Patienten, bei dem diesbezüglicher Bedarf besteht, gefolgt von der Aufnahme eines MRI-Bilds des Patienten.

16. Verfahren zur enantioselektiven Synthese der Verbindung der Formel (IA) nach Anspruch 1, umfassend:
(a) Monoalkylierung einer Verbindung der Formel (III) unter Verwendung eines ersten Enantiomers von 2-(Methylsulfonyloxy)pentandisäure-5-benzylester-1-tert-butylester 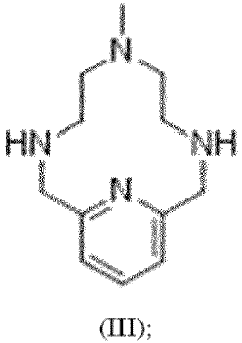
(b) gefolgt von Alkylierung der Verbindung aus Schritt (a) mit einem zweiten Enantiomer von 2-(Methylsulfonyloxy)pentandisäure-5-benzylester-1-tert-butylester, wobei das zweite Enantiomer dem ersten Enantiomer entgegengesetzt ist;
(c) Umsetzen des Produkts aus Schritt (b) mit Mn; und
(d) Umsetzen des Produkts aus Schritt (c) mit einem Aminoalkohol.

17. Verfahren zur enantioselektiven Synthese einer Zusammensetzung, die Formel (IA) umfasst, nach Anspruch 4, umfassend:
(a) Monoalkylierung einer Verbindung der Formel (III) unter Verwendung eines ersten Enantiomers von 2-(Methylsulfonyloxy)pentandisäure-5-benzylester-1-tert-butylester 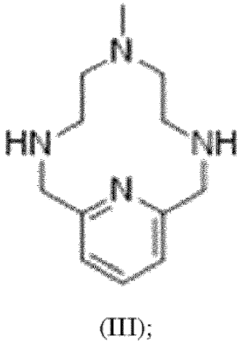
(b) gefolgt von Alkylierung der Verbindung aus Schritt (a) mit einem zweiten Enantiomer von 2-(Methylsulfonyloxy)pentandisäure-5-benzylester-1-tert-butylester, wobei das zweite Enantiomer dem ersten Enantiomer entgegengesetzt ist;
(c) Umsetzen des Produkts aus Schritt (b) mit Mn; und
(d) Umsetzen des Produkts aus Schritt (c) mit einem Aminoalkohol.

18. Verfahren zur Herstellung der Zusammensetzung nach Anspruch 4, wobei das Verfahren Folgendes umfasst:
Erhitzen einer Zusammensetzung, die die Verbindung der Formel (IB): 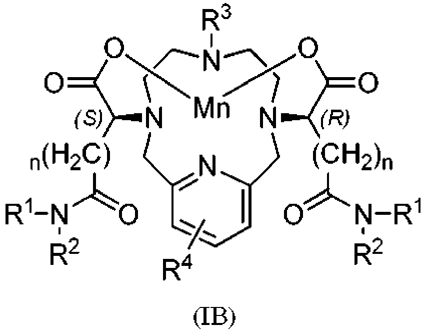
oder ein Salz oder Solvat davon umfasst;
wobei das Erhitzen über einen ausreichenden Zeitraum und bei einer ausreichenden Temperatur zur weitgehend vollständigen Umwandlung der Verbindung der Formel (IB) in die Verbindung der Formel (IA) erfolgt, gegebenenfalls wobei die resultierende Zusammensetzung keine nachweisbaren Mengen der Verbindung der Formel (IB) enthält.

## Revendications

1. Composé de formule IA : 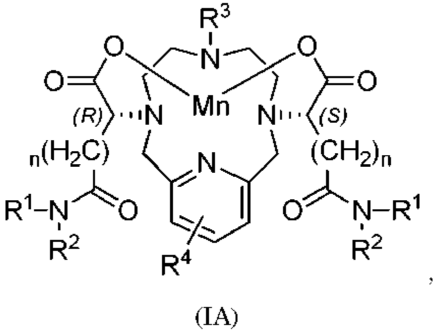 ou sel ou solvate correspondant :
chaque R¹ étant indépendamment choisi parmi C₁₋₂₀ hydroxyalkyle, C₁₋₆ alkyle, C₃₋₆ aryle éventuellement substitué par un ou plusieurs substituants choisis parmi halogéno et -C (=O)-NH-C₁₋₆ hydroxyalkyle, ou un groupement glucide ;
chaque R² étant indépendamment choisi parmi C₁₋₂₀ hydroxyalkyle, C₁₋₆ alkyle ou hydrogène ;
R³ étant choisi parmi C₁₋₃ alkyle ou -(CH₂)ₘ-C(=O)- NR⁵R⁶, m étant un entier de 2-5, R⁵ et R⁶ étant indépendamment choisis parmi hydrogène, parmi C₁₋₂₀ hydroxyalkyle, C₁₋₆ alkyle, C₃₋₆ aryle éventuellement substitué par un ou plusieurs substituants choisis parmi halogéno et -C(=O)-NH-C₁₋₆ hydroxyalkyle, ou un groupement glucide ;
R⁴ représentant 0-3 substituants choisis parmi hydroxy, C₁₋₆ alkyle et C₁₋₆ hydroxyalkyle ; et,
chaque n étant un entier de 0-4.

2. Composé selon la revendication 1, le composé étant de formule IA,
chaque R¹ étant C₁₋₂₀ hydroxyalkyle ;
R³ étant méthyle ;
R² et R⁴ étant hydrogène ; et
n étant 2.

3. Composé ayant la structure :

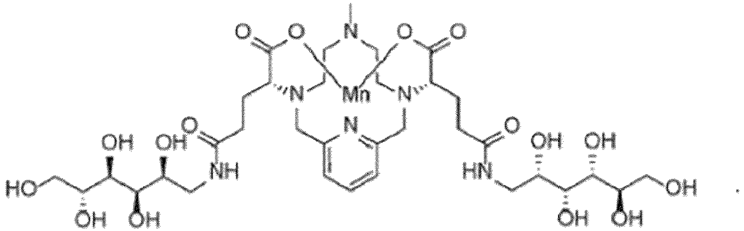

4. Composition comprenant un composé de formule IA : 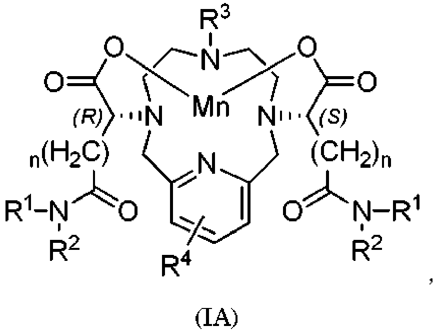 ou un sel ou un solvate correspondant, et un excipient pharmaceutiquement acceptable :
chaque R¹ étant indépendamment choisi parmi C₁₋₂₀ hydroxyalkyle, C₁₋₆ alkyle, C₃₋₆ aryle éventuellement substitué par un ou plusieurs substituants choisis parmi halogéno et -C(=O)-NH-C₁₋₆ hydroxyalkyle, ou un groupement glucide ;
chaque R² étant indépendamment choisi parmi C₁₋₂₀ hydroxyalkyle, C₁₋₆ alkyle ou hydrogène ;
R³ étant choisi parmi C₁₋₃ alkyle ou -(CH₂)ₘ-C(=O)- NR⁵R⁶, m étant un entier de 2-5, R⁵ et R⁶ étant indépendamment choisis parmi hydrogène, parmi C₁₋₂₀ hydroxyalkyle, C₁₋₆ alkyle, C₃₋₆ aryle éventuellement substitué par un ou plusieurs substituants choisis parmi halogéno et -C(=O)-NH-C₁₋₆ hydroxyalkyle, ou un groupement glucide ;
R⁴ représentant 0-3 substituants choisis parmi hydroxy, C₁₋₆ alkyle et C₁₋₆ hydroxyalkyle ; et,
chaque n étant un entier de 0-4.

5. Composé selon la revendication 1 ou composition selon la revendication 4, chaque R¹ étant :
(i) C₁₋₂₀ hydroxyalkyle ; ou
(ii) indépendamment C₃₋₉ hydroxyalkyle ; ou
(iii) indépendamment C₃₋₆ hydroxyalkyle.

6. Composé selon la revendication 1 ou composition selon la revendication 4, R³ étant méthyle.

7. Composé selon la revendication 1 ou composition selon la revendication 4, R² et R⁴ étant hydrogène.

8. Composé selon la revendication 1 ou composition selon la revendication 4, n étant 2.

9. Composé selon la revendication 1 ou composition selon la revendication 4, le composé de formule IA comprenant au moins deux groupes hydroxy.

10. Composition selon la revendication 4, le composé de formule IA ayant la structure :

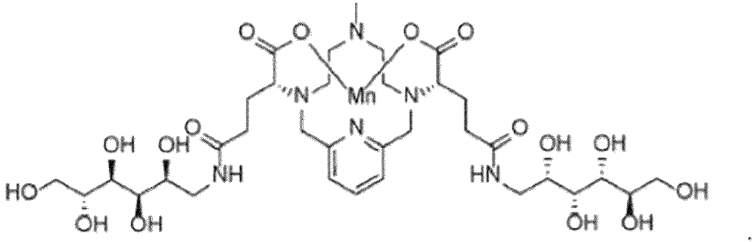

11. Composition selon la revendication 4, la composition étant dépourvue de quantités détectables du composé de formule (IB) : 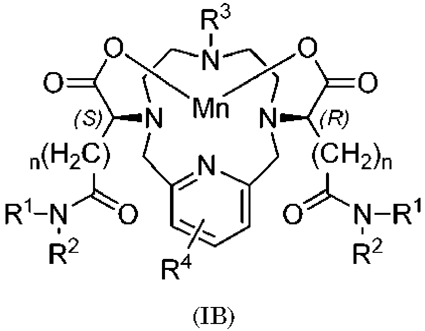 ou un sel ou un solvate correspondant.

12. Composition selon la revendication 11, le composé de formule (IB) ayant la structure :

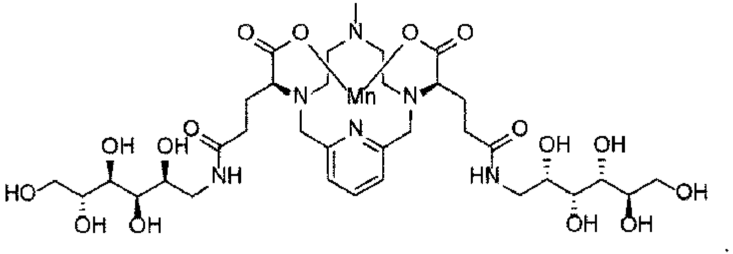

13. Composition selon la revendication 4, la composition comprenant en outre des chélates de Mn ayant une stéréochimie (R,R) et (S,S).

14. Procédé d'imagerie d'un patient comprenant une administration du composé selon la revendication 1 ou la revendication 3 à un patient qui en a besoin, suivie par l'acquisition d'une image d'IRM du patient.

15. Procédé d'imagerie d'un patient comprenant une administration de la composition selon la revendication 4 ou la revendication 10 à un patient qui en a besoin, suivie par l'acquisition d'une image d'IRM du patient.

16. Procédé de synthèse énantiosélective du composé de formule (IA) selon la revendication 1 comprenant :
(a) une monoalkylation en utilisant un premier énantiomère de 2-(méthylsulfonyloxy)pentanedioate de 5-benzyle et de 1-tert-butyle d'un composé de formule (III) 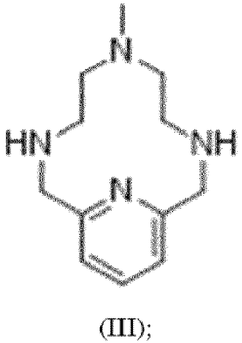
(b) suivie par une alkylation du composé provenant de l'étape (a) avec un deuxième énantiomère de 2-(méthylsulfonyloxy)pentanedioate de 5-benzyle et de 1-tert-butyle, le deuxième énantiomère étant l'opposé du premier énantiomère ;
(c) la mise en réaction du produit de l'étape (b) avec Mn ; et
(d) la mise en réaction du produit de l'étape (c) avec un aminoalcool.

17. Procédé de synthèse énantiosélective d'une composition comprenant la formule (IA) selon la revendication 4 comprenant :
(a) une monoalkylation en utilisant un premier énantiomère de 2-(méthylsulfonyloxy)pentanedioate de 5-benzyle et de 1-tert-butyle d'un composé de formule (III) 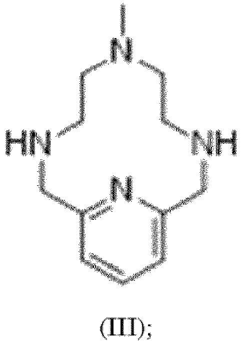
(b) suivie par une alkylation du composé provenant de l'étape (a) avec un deuxième énantiomère de 2-(méthylsulfonyloxy)pentanedioate de 5-benzyle et de 1-tert-butyle, le deuxième énantiomère étant l'opposé du premier énantiomère ;
(c) la mise en réaction du produit de l'étape (b) avec Mn ; et
(d) la mise en réaction du produit de l'étape (c) avec un aminoalcool.

18. Procédé de préparation de la composition selon la revendication 4, le procédé comprenant :
le chauffage d'une composition comprenant le composé de formule (IB) : 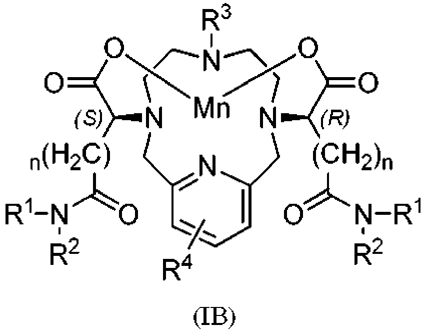
ou un sel ou un solvate correspondant ;
le chauffage étant pendant une durée et une température suffisantes pour convertir sensiblement tout le composé de formule (IB) en le composé de formule (IA), éventuellement
la composition résultante étant dépourvue de quantités détectables du composé de formule (IB).
